(19) Europäisches Patentamt
European Patent Office
Office européen des brevets

(11) **EP 3 549 139 B1**

(12) # EUROPEAN PATENT SPECIFICATION

(45) Date of publication and mention
of the grant of the patent:
**03.06.2026 Bulletin 2026/23**

(21) Application number: **17808401.8**

(22) Date of filing: **27.11.2017**

(51) International Patent Classification (IPC):
**G16H 50/30** (2018.01)　　**G16H 20/60** (2018.01)

(52) Cooperative Patent Classification (CPC):
**G16H 20/60; G16H 50/30**

(86) International application number:
**PCT/EP2017/080451**

(87) International publication number:
**WO 2018/099838 (07.06.2018 Gazette 2018/23)**

(54) **SYSTEM AND METHODS FOR CALCULATING, DISPLAYING, MODIFYING, AND USING IMPROVED PERSONALIZED NUTRITIONAL HEALTH SCORE TO ASSESS AND PLAN OPTIMAL DIETS**

SYSTEM UND VERFAHREN ZUR BERECHNUNG, ANZEIGE, MODIFIZIERUNG UND VERWENDUNG EINES VERBESSERTEN PERSONALISIERTEN ERNÄHRUNGSGESUNDHEITSWERTE ZUR BEURTEILUNG UND PLANUNG OPTIMALER DIÄTEN

SYSTÈME ET PROCÉDÉS POUR CALCULER, AFFICHER, MODIFIER ET UTILISER UN SCORE DE SANTÉ NUTRITIONNELLE PERSONNALISÉ AMÉLIORÉ POUR ÉVALUER ET PLANIFIER DES RÉGIMES OPTIMAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2016 US 201662429377 P**

(43) Date of publication of application:
**09.10.2019 Bulletin 2019/41**

(73) Proprietor: **Société des Produits Nestlé S.A.**
**1800 Vevey (CH)**

(72) Inventor: **MAINARDI, Fabio**
**1180 Rolle (CH)**

(74) Representative: **Chautard, Cécile**
**Société des Produits Nestlé S.A.**
**Avenue Nestlé 55**
**1800 Vevey (CH)**

(56) References cited:
**WO-A1-2016/050958**

- **MAINARDI FABIO ET AL: "Personalized Nutrient Profiling of Food Patterns: Nestlé's Nutrition Algorithm Applied to Dietary Intakes from NHANES", NUTRIENTS, vol. 11, no. 2, 12 February 2019 (2019-02-12), pages 379, XP055941092, DOI: 10.3390/nu11020379**
- **JENNIFER J OTTEN; LINDA D MEYERS; JENNIFER PITZI HELLWIG: "Dietary Reference Intakes: The Essential Guide to Nutrient Requirements.", 31 December 2006, THE NATIONAL ACADEMIES PRESS, Washington, DC, pages: 1 - 82(68), XP055450504, DOI: https://doi.org/10.17226/11537**
- **ANONYMOUS: "Dietary Reference Intake - Wikipedia", 15 October 2016 (2016-10-15), XP055450501, Retrieved from the Internet <URL:https://web.archive.org/web/20161015222358/https://en.wikipedia.org/wiki/Dietary_Reference_Intake> [retrieved on 20180212]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

# EP 3 549 139 B1

**Description**

COPYRIGHT NOTICE

TECHNICAL FIELD

The present disclosure relates in general to systems and methods for calculating the impact of dietary intakes on the nutritional health of populations. More specifically, the present disclosure relates to systems and methods for determining whether the nutritional content of a particular food item, such as an ingredient, a food, a meal, or a diet, falls within a plurality of so-called healthy ranges customized to an individual, and calculates scores based on those determinations. The present disclosure further accounts for upper and lower endpoints, or limits, on nutrient intakes, such that in addition to determining recommended intake, a lower and an upper bound for nutrient intake are determined. For example, the present disclosure in some embodiments advantageously relies on Estimated Average Requirement (EAR) and Recommended Dietary Allowance (RDA) to determine an appropriate minimum amount of a consumable for an individual to consume.

BACKGROUND

Over the past century, nutrient deficiencies have generally decreased, especially in the developed countries, and infectious disease rates have dropped. At the same time, the rates of chronic, diet-related diseases have risen. National surveys (*e.g.*, the National Health and Nutrition Examination Survey in the US) reveal poor quality eating patterns. Cardiovascular disease, high blood pressure, type 2 diabetes, some cancers, and poor bone health have been shown to have some degree of correlation with such eating patterns. The proportion of overweight or obese people is increasing. It is important to realize that these poor eating patterns correspond to excessive consumption of certain nutrients, like saturated fat or sugar, but also to low intake of other important nutrients, like vitamin B6 or folate.

Making healthier food choices can help prevent non-communicable diseases such as obesity, cardiovascular disease, diabetes and some cancers. Guidelines provide general population recommendations for healthful eating. Such guidelines can drive content for nutrition labels, health claims, nutrition education, menu planning, as well as marketing and advertising on food products. Recommendations for healthier food choices are generally promulgated in two levels of granularity: at the level of food groups (*e.g.*, eat more fruits, eat more whole grains) or at the nutrient level. However, these generalized guidelines are not designed to provide personalized nutrition recommendations at the macro- or micronutrient level.

Substantial efforts have been made to quantify and track the impact of particular consumables, such as ingredients, foods, meals, or diets, on the overall health of individuals. For example, every five years since 1980, the United States Department of Agriculture (USDA) along with the Department of Health and Human Services (HHS) releases so-called Dietary Guidelines for Americans. The USDA states that these Guidelines provide advice about consuming fewer calories, making informed food choices, and being physically active to attain and maintain a healthy weight. (http://www.cnpp.usda.gov/DietaryGuidelines).

Likewise, many other national and international agencies have published recommendations for daily intake of nutrients, for apparently healthy individuals of all ages. These recommendations are applied in many contexts, including: composition of diets for schools, prisons, hospitals or nursing homes; development of new food products in the industry; and decision making by health-care policy makers and public health officials.

To this end, the Institute of Medicine (IOM) in the US has created a general framework called the Dietary Reference Intakes (DRI); this reference framework has been subsequently adopted by other countries. Under the IOM's framework, for a given nutrient for which adequate knowledge is available, a set of DRIs is defined as follows:

- Estimated Average Requirement (EAR): the average daily nutrient intake level estimated to meet the requirement of half the healthy individuals in a particular life stage and gender group
- Recommended Dietary Allowance (RDA): the average daily nutrient intake level sufficient to meet the nutrient requirement of nearly all (*e.g.*, 97 to 98 percent) healthy individuals in a particular life stage and gender group
- Tolerable Upper Level (UL): the highest average daily nutrient intake level likely to pose no risk of adverse health effects to nearly all individuals in the general population; as intake increases above the UL, the potential risk of adverse effects increases

- Adequate Intake (AI): a recommended average daily nutrient intake level based on observed or experimentally determined approximations or estimates of nutrient intake by a group (or groups) of apparently healthy people that are assumed to be adequate; it is used when an RDA cannot be determined

While the DRIs discussed above provide a general framework into which individuals can seek to fit themselves, they are nonetheless an inadequate tool to enable individuals to track the actual impact of consumables on overall nutritional health. Specifically, because these mechanisms are nothing more than guidelines, it is difficult for individuals to determine the actual nutritional goals they should be attempting to achieve, and whether/when those goals have been achieved. That is, it is difficult for individuals to determine healthy amounts of particular nutrients that should be consumed over a given time period and to track whether those healthy amounts have in fact been consumed.

While certain known tools may enable users to track nutrient intake, such known tools do not provide the capability to measure the nutrient intakes from consumed food, score them, and compare the nutrient intake with DRIs to provide guidance for improvement.

Moreover, the endpoints defining the criterion of nutritional adequacy or adverse effects depend on the nutrient and on the life stage and gender of the individual to whom the endpoints apply. Certain of the mechanisms described above lack this granularity.

To assist nutrition professionals and individuals in navigating more specific nutrition intake goals, food scientists have attempted to develop scoring systems to rate the healthfulness or unhealthfulness of foods, meals, and diets. However, reviewers have noted these are often methodologically weak. In certain existing schemes that attempt to apply scores to foods, a single score is determined and applied to the food itself without consideration of the individual consuming the food or the amount of the food consumed. This is ineffective, as the nutritional health of a given food depends both on the individual consuming the food (*e.g.*, the caloric or other nutritional needs of the individual) and the amount of the food consumed (*e.g.*, a half-cup of ice cream versus a half-gallon of ice cream).

Most nutritional scores according to known systems use a single recommendation for the minimal intake of each nutrient, usually the RDA, based on the assumption that it is the most protective value in the sense of covering the requirements for almost all the population. The limitation of this approach is that the calculated nutritional score is tailored to the most sensitive individuals in the population, and does not reflect the fact, recognized in the instant disclosure, that the actual requirement for a given individual is more likely to track the EAR.

It has been argued by some authors, including George H. Beaton ("Choice of DRI Value for Use in Nutrition Labeling," Journal of Nutrition, 2007), that the EAR would be a better choice as a nutritional score. However, using only the EAR may not be satisfactory either, as this approach utilizes only part of the information provided by the conceptual framework defining the DRI.

A specific example of some of the weaknesses in an approach using either the EAR or RDA values alone can be seen by an example. For a female in the age group 31-50, not pregnant or lactating, the Institute of Medicine provides the following DRIs for Vitamin D:

- 

$$EAR = 625 \mu g/day$$

- 

$$RDA = 900 \mu g/day$$

- 

$$UL = 3000 \mu g/day$$

These numbers define a set of "healthy intakes" of Vitamin D, ranging roughly from 900 to 3000 $\mu$g/day. The endpoints of this interval are fuzzy and must be interpreted in probabilistic terms, because an intake slightly below 900 is very likely to be adequate for a given individual, while a single intake of 3500 $\mu$g/day is unlikely to cause any adverse effect to a healthy individual. However, because of the possibility that 3500 $\mu$g/day intake could be harmful over time for some individuals, even the use of such "fuzzy" endpoints with such a model is insufficient because it fails to take account of that fact. In other words, using either the EAR or the RDA alone as a single point from which to derive a nutritional score neglects the information about the inter-individual variability of the requirements. Such approaches are thus inadequate to calculate a usable nutritional score.

Existing systems and schemes have typically sought to classify nutrients as either so-called "qualifying" nutrients or so-called "disqualifying" nutrients. In general, qualifying nutrients have been viewed as having a lower limit, such that exceeding the lower limit is viewed as "good" and being below the lower limit is viewed as "bad." Similarly, disqualifying nutrients have been viewed as having an upper limit, such that remaining below the upper limit has been generally viewed as "good" while exceeding the upper limit has generally been viewed as "bad." These schemes are inadequate because they fail to provide a common mechanism for tracking the impact on all kinds of nutrients (or other measurable aspects of foods) that can account for overconsumption where appropriate, and do not need to define overconsumption if a particular nutrient does not demand it.

Another important difference with many existing measures (including the Healthy Eating Index) is that they rely on *density* measures, meaning that the limits used to score dietary components are defined with respect to caloric intakes (*e.g.*, per 1000 kilo-calories). This means that the score is independent of the actual amounts consumed (multiplying all the amounts by the same factor does not change the value of the index). However, dietary reference intakes are generally defined as absolute amounts for a given period of time, and most such definitions are not directly related to the energy intake.

In other known systems, nutritional balance scores have been utilized to drive indications of the completeness of the nutrients consumed. These systems may utilize DRI values but only score a subset of nutrients (typically vitamins and minerals) with a desired minimal limit. The global completeness of consumed vitamins and minerals can be modified that a score between 0 and 100 is given, where 100 is given if all vitamins and minerals are consumed above their limit and some fraction of 100 given based on the under-consumption of nutrients. These scoring systems do not take into account healthy ranges and are not capable of accounting for upper limits beyond which consumption is unhealthy. Many vitamins and minerals have toxic levels of consumption that are unhealthy and these systems cannot account for unhealthy high doses. Additionally, nutrients like fats and sugars that may be beneficially limited are not taken into account. Finally, and importantly, the utilization of only lower limit thresholds to produce nutritional scores results in scores that cannot be optimized as a function of consumption, because the score only increases as consumption increases.

Known systems and schemes are also deficient because they are not constructed at an appropriate level of granularity to improve scoring for heterogeneous populations or individuals. Instead, one set of values is used to define a single score for populations and all individuals. This lack of granularity prohibits known systems and schemes from being customized to different individual users with different individual nutritional needs. The system known from WO2016050958A1 calculates a single score for a consumable that indicates the nutritional health of that consumable to indicate whether nutrients within a consumable are within a healthy range based on a recommended intake. This known system determines whether the content of a consumable falls within a range customized to the user, and then it generates nutritional advice on the impact of changes to nutritional habits on the user's overall nutritional health. -The paper titled "Dietary Reference Intakes: The Essential Guide to Nutrient", authored by Jennifer J Otten; Linda D Meyers; Jennifer Pitzi Hellwig: published by The National Academies Press, Washington, DEPENDENT CLAIM on 31 December 2006, https://doi.org/10.17226/11537 (XP055450504) discusses the Dietary Reference Intakes (DRIs) for setting nutrient values and for assessing and planning diets for individuals. -The Wikipedia article titled "Dietary Reference Intake" published on 15 October 2016 (XP055450501) and available online at https://web.archive.org/web/20161015222358/https://en.wikipedia.org/wiki/Dietary Refe rence Intake also presents the Dietary Reference Intakes (DRIs) and some application thereof.

What is needed is a system that uses a combination of certain developed minimum intake amounts to derive a minimum intake amount of a nutrient for an individual that more closely reflects that person's nutritional situation and needs.

What is further needed is a system that calculates a plurality of values for this nutritional health score taking into account nutrient requirements specific to a particular individual, such that the score is customized to the individual for different amounts of foods consumed.

What is further needed is a system that calculates customized nutritional health scores based on adjustable sets of nutrients and adjustable weights/tolerance values to design score profiles for a particular use case or purpose, such as for performance in athletics.

What is still further needed is a system that can calculate the impact of either adding or removing consumables on the individual's overall nutritional health score, such that the system can make recommendations of additional consumables the individual can consume (or can remove from his or her diet) to ensure that all necessary nutrients are consumed in healthy amounts for a given period, such as for a given day or week.

What is still further needed is a system that can determine useful endpoints for intake of nutrients, such that a minimal and maximal amount of each nutrient (whether "good" or "bad") can be determined and used to assess overall nutritional health through a score reflective of these endpoints.

The present disclosure describes a nutritional health scoring system that satisfies the needs described above. Thus, the present disclosure describes a system and methods that overcome the shortcomings of prior nutritional management techniques described above.

SUMMARY

The present invention is set out in the appended claims. The instant disclosure is based on the distribution of nutrient requirements within a population, whenever this information is available. In various embodiments, the score calculated by the disclosed methodology can be interpreted as a utility function and techniques from economic analysis and game theory can then be applied to the context of nutrition and dietary planning. Accordingly, it is believed that the system and methods disclosed herein can be used as a valid and reliable indicator of how balanced and adequate a diet is with respect to typical nutritional needs.

In various embodiments, the system and methods disclosed herein assess the quality of diets using the full knowledge and distributional properties of the DRI or other intake recommendation regimes. In some embodiments, the disclosed system and method takes into account "upper" and "lower" limits of intake recommendations for each nutrient, regardless of whether that nutrient is generally characterized as "good" or "bad." In particular, embodiments of the system disclosed herein use a combination of EAR and RDA. The disclosed system can then determine a personalized, minimum intake amount for an individual over a given period of time, and calculate nutritional health scores based on this amount. In this way, in some embodiments, the system and methods disclosed herein enable measurement of how far actually-consumed nutrient amounts are from dietary reference intakes.

In some embodiments, the distance from the reference intakes can be further used to assess overall nutritional health, as described in detail below. This enables the system and methods disclosed herein to more closely adhere to the recommendation, from various Institute of Medicine reports, that greater emphasis is to be placed on the distribution of nutrient requirements within a population, rather than on a single value and that the concepts of probability and risk explicitly underpin the determination of the DRIs and inform their application in assessment and planning.

In various embodiments, the system and methods disclosed herein derive and use absolute amounts for nutrient intakes (upper and lower limits as well as recommended intakes) where the recommendations for that nutrient are expressed (*e.g.*, in the DRI) in units of absolute mass.

Embodiments of the disclosed system offer a variety of software and analytic tools to assess, plan, and optimize diets on a person-by-person (or population group-by-population group) basis, and take into account both recommended intake amounts and endpoints for minimum and maximum intake amounts. In various embodiments, the disclosed system determines the nutritional adequacy, in terms of minimum amounts to be consumed.

In some embodiments, the system and methods disclosed herein can be used by nutritionists, health-care professionals, and individual users (*e.g.*, users of wearable devices such as smart watches or fitness trackers). In an exemplary embodiment, the system disclosed herein includes at least one processor configured to execute an algorithm to calculate a single score for measuring the nutritional balance of meals and diets. In this embodiment, the algorithm takes into account factors specific to an individual (or population of individuals) and determines the score based on a plurality of nutrients within that individual's (or population's diet). In an embodiment, "nutritional balance" is a term used with respect to general dietary recommendations, primarily the dietary reference intakes from the Institute of Medicine.

In various embodiments, the user-specific (or population-specific) inputs to the disclosed system are programmable and configurable, and include gender, age, weight, height physical activity level, whether pregnant or lactating, and the like.

In some embodiments, the system disclosed herein is configured to evaluate adequacy of nutrient intake in terms of maximal amounts. In these embodiments, the system takes into consideration toxicity and adverse effects of consuming too much of a particular nutrient or consumable. In some such embodiments, only those nutrients or consumables with a recognized harmful maximal intake level (*i.e.*, those items where consuming too much can be harmful) are taken into account when calculating an overall health score. In other embodiments, nutrients without a recognized harmful maximal intake level are used to determine an overall health score; for these nutrients, the individual nutrient health score does not decrease as more of the nutrient is consumed, reflecting the fact that there is no recognized maximal amount.

In an embodiment, the disclosed system includes or is connected to a database containing foods or food composition items and respective nutrient content. In this embodiment, the disclosed system includes a fuzzy search feature that enables a user to enter a consumed (or to-be consumed) food, and thereafter searches the database to find a closest item to the user-provided item. The disclosed system, in this embodiment, uses stored nutritional information about the matched food item to determine a score as described below.

In various embodiments, the disclosed system further includes an interface (e.g., a graphical user interface) to display the amount of each nutrient available in each food composing the diet. In some embodiments, this interface enables users to modify the amount of various foods to be consumed, and correspondingly displays a health score based on the modified amount of food to be consumed. In other embodiments, the system is configured to determine amounts of food consumed using non-user-input data, such as by scanning one or more bar codes, QR codes, or RFID tags, or by tracking items ordered from a menu or purchased at a grocery store.

In some embodiments, the system and methods disclosed herein are configured to calculate adequacy of nutritional content of food for a particular individual or group of individuals. In these embodiments, "adequacy" of the nutritional value of a food or other consumable is based on a user profile including information such as gender, age, body measurements, and other health-related conditions like pregnancy or lactation.

In some embodiments, the nutrient score for a particular nutrient whose intake sufficiency is defined in terms of an Adequate Intake (AI) value is calculated as the smaller of a maximum score and the percentage of the AI value consumed by the individual. Thus, the system disclosed herein can calculate scores for nutrients even where the nutrient does not have established EAR and RDA values.

The disclosed system includes a recommendation feature that recommends particular foods to an individual (or population) that will maximize the overall nutritional balance of the individual (or population). In such embodiments, an algorithm executed by the disclosed system generates a list of recommendations to improve the nutritional balance and can be a valuable tool for nutritionists for the planning and assessment of a diet.

In various embodiments, the system disclosed herein calculates one or more nutritional health scores tailored to an individual based on the individual's caloric intake range and corresponding healthy ranges of nutrient intakes for a given time period. The calculated scores are based on whether nutrient intake falls within a healthy range, and are affected not only by under-consumption of nutrients but also by over-consumption of nutrients. These scores enable individuals to determine whether they are consuming enough nutrients, and to the extent they are not, to determine which additional nutrients need to be consumed. The disclosed system also makes suggestions for adding or removing consumables that, if consumed (or removed from a diet), will provide the individual with nutrients in amounts determined to be within healthy nutrient ranges for that individual.

In various embodiments, the nutritional health scores calculated by the disclosed system indicate the nutritional health of the individual for whom the scores are calculated. Nutritional health as used in these embodiments refers to the extent to which nutrients consumed by an individual are within the individual's healthy nutrient ranges over a specified period of time. In these embodiments, exemplary nutrients can include micro-nutrients (*e.g.*, calcium, cholesterol, fiber) and/or macro-nutrients (*e.g.*, carbohydrates, protein, and saturated fat). The types of nutrients that can be assessed using the disclosed system are discussed in further detail below.

Various embodiments of the disclosed system are based on the premise that all nutrients have healthy ranges for consumption. That is, embodiments of the disclosed system are based on the premise that there are no good or bad nutrients, and hence no intrinsically good or bad foods. Instead, for each nutrient (or food), a person either consumes an amount that is inside or outside a healthy range for consumption. In these embodiments, the healthy ranges of nutrients can be different for different individuals, meaning that an assessment of nutritional health depends on the needs of a specific individual. For example, the healthy ranges of particular nutrients can vary for different people depending on whether a person is pregnant or lactating, whether a person has diabetes, whether a person is obese, whether a person is a critical care patient, whether a person has allergies, or whether a person is an athlete. As described below, by varying the healthy range for different nutrients in a way that is customized to the person, the calculated nutritional health score provided by the disclosed system is also customized to each individual user.

Various embodiments of the disclosed system display a dashboard or other appropriate user interface to a user that is customized based on the user's nutritional needs, such as the user's caloric intake or a set of determined applicable DRI (daily reference intake) values. The disclosed system calculates scores indicative of the nutritional value of a consumable, such as an ingredient, a food, a meal, or a diet, and displays the scores to the user via the dashboard. In these embodiments, the calculated scores are functions of the amount of food (and therefore nutrients) consumed over a given time period and are also tailored to an individual user such that the scores indicate the nutritional value of the consumable to a single, particular individual as opposed to indicating the general nutritional value of a consumable to a group of individuals. In such embodiments, the disclosed system calculates component nutrient health scores by determining whether nutrient content of a consumable is within a range tailored to the user for each nutrient contained in the consumable. The system then composes or aggregates the component scores into aggregate scores based on a personalized set of weighting parameters and/or sensitivity values ascribed to each nutrient that reflect the overall nutritional health impact of the consumable for the individual. In various embodiments, therefore, it can be said that the nutritional health scores represent the extent to which nutrient consumption is within defined nutritional health ranges as a weighted average of individual nutrient scores outside nutrient healthy ranges.

In various embodiments, the disclosed system is configured to calculate and display multiple nutritional health scores to provide a complete picture of the nutritional impact of consuming certain consumables. In one embodiment, the scores are calculated according to an equation that takes into account the amount of the food consumed over a given time period and also characteristics of the individual for whom the scores are calculated. In one embodiment, for a particular individual, multiple scores could be given for different purposes. In one embodiment, the disclosed system calculates at least two different scores for a given consumable: one score indicating the nutritional content of the current or actual amount of food consumed, and one score that is a highest possible score for that food, where the amount of food consumed for a set period of time is variable. Thus, for example, the system may provide an indication that since an individual consumed 1/4 pound of chicken in a day, his score is X, but his score for chicken would be at a maximum value of Y>X if he consumed an additional 1/4 pound of chicken in the day. In other words, the disclosed system can provide the score of a meal as built, and can provide an optimal score that might be achieved if additional food items are consumed or if certain consumed foods are removed or reduced from a diet.

Various embodiments of the disclosed system also provide an advisory functionality. In these embodiments, after calculating a nutritional health score for a particular individual based on ranges that define that individual's nutrient and caloric needs for a given time period (*e.g.*, a given day), the disclosed system suggests combinations of consumables that can be consumed for the remainder of the time period to result in the individual obtaining the nutrients he or she requires. For example, if an individual indicates that he or she has eaten certain foods for breakfast and lunch, the disclosed system can suggest a dinner menu that will ensure the individual gets all the nutrients he or she needs in the day while still consuming an amount of calories that falls within a caloric intake range applicable to the individual. In this embodiment, the recommendations provided by the disclosed system are optimized; the system determines the impact on the overall nutritional health score of a plurality of foods stored in its database, and suggests foods that result in an optimal increase to the nutritional health score.

In one embodiment, the system determines the total nutrient content of the consumable or group of consumables before determining a nutrient health score for each nutrient. In this embodiment, the nutrient health score for each nutrient is less than 1 (or some other maximum) if the nutrient content is outside the range for that individual, and is 1 (or some other maximum) if the nutrient content is in the range for that individual. The amount by which the nutritional health score differs from 1 (or some other maximum) indicates the extent to which the nutrient in a consumable is outside the range determined to be ideal for an individual. This scoring calculation also takes into account both the amount by which a nutrient is under-consumed (*i.e.*, is consumed in amount less than a healthy range for the nutrient) and an amount by which the nutrient is over-consumed (*i.e.*, is consumed in an amount greater than the healthy range for the nutrient).

Given the component nutritional health scores for the individual nutrients of a consumable, the disclosed system further calculates an aggregate nutritional health score by computing a weighted average of the scores for the nutrients. In various embodiments, this is done by assigning a weighting value to each nutrient in the scoring profile, multiplying the nutritional health score for that nutrient by the weight, and summing the scores of all the scores of the nutrients in the consumables. In an embodiment, the weighting scores sum to 100. As a result, the overall nutritional health score in this embodiment will be a number less than or equal to 100. If the component nutritional health scores for each nutrient in the consumable are each 1 (meaning that each nutrient of the consumable is within the healthy range for the individual), the overall nutritional health score will be 100 (*i.e.*, the sum of the weights of the nutrient components). Thus, in one embodiment, a score of 100 indicates that each of the individual's nutrient requirements are being met, and a number less than 100 indicates they are not, with the difference representing an amount by which the nutrient needs are not being met.

It should thus be appreciated that the disclosed system provides the advantage over known systems in that the particular food consumed does not have a single, static score, but rather has a scoring profile or function that is tailored to an individual that can be used to determine scores for the food under different conditions, such as different caloric intake requirements or different amounts of food consumed. It should be further appreciated that in calculating the scoring profile, the disclosed system is advantageous in that it takes into account endpoints that represent both a minimal and maximal value that should be consumed by an individual for the individual not to do harm to their overall health by consuming less than the minimal amount or more than the maximal amount.

In various embodiments, the disclosed system stores some or all of the values needed to calculate nutritional health scores in one or more databases. For example, the disclosed system may store a table of caloric intake ranges for individuals based on the age, gender, and weight or Body Mass Index (BMI) of the individuals. In this embodiment, to determine an individual's caloric intake range for a given time period, the individual must provide the system with his or her age, gender, and weight or BMI. By performing a database lookup or calculation, the disclosed system can thus determine a caloric intake range for a given time period for a given individual.

In one embodiment, the disclosed system enables a user to customize the nutritional health score to suit him or her by indicating his or her age, gender, and weight/BMI. This affects the caloric intake range for the individual, and thus affects the lower and upper healthy range values for each nutrient tracked by the system. In another embodiment, the disclosed system provides for further customization by enabling the user to specify additional information, such as body type, physical activity level, and the like. In this embodiment, the disclosed system uses these additional inputs to adjust not only optimal caloric intake ranges for different individuals, but also lower and upper healthy range values for nutrients tracked by the system. For example, if an individual indicates that he or she is athletic with a relatively high amount of athletic activity, the system may adjust the carbohydrate nutrient range upward to account for the individual's need for additional carbohydrates.

Accordingly, various embodiments of the disclosed system advantageously enable the calculation of a nutritional health score for an individual by performing the following steps:

> (1) Storing indications of a plurality of nutrients to be scored
> (2) Storing indications of healthy ranges for each of the stored nutrients, including lower intake limits determined by
> combining various recommendations from various appropriate organizations in a way specific to an individual

(3) Storing indications of endpoints for nutrient consumption to enable the system to adjust for over- and under-consumption beyond the endpoints, as is appropriate for each nutrient

(4) Storing score weighting and individual tolerance values for each nutrient and/or individual

(5) For a particular consumable, compute a nutrient health score for each component nutrient

(6) Compute the aggregate nutritional health score for the consumable by applying weight values for each nutrient

Various embodiments of the disclosed system further advantageously provide nutritional advice to users based on calculated nutritional health scores. For example, embodiments of the disclosed system determine amounts of nutrients that would be needed to place an individual in the healthy amount range for those nutrients. These embodiments then analyze a database of consumables (e.g., foods or ingredients) to determine combinations of consumables that will provide the needed amounts of nutrients to place the user in the healthy amount ranges while still remaining within the optimal caloric intake range for that individual.

The disclosed system works in conjunction with a laboratory or other testing facility that generates actual data about individuals using the disclosed system. For example, in one embodiment the disclosed system enables a user to submit a blood spot test to determine the makeup of the individual's blood, including whether the individual is over- or under-consuming various nutrients. This testing and lab work enables the system to verify that its recommendations are working-that is, to verify that a user is actually receiving adequate nutrients when the scoring function indicates that his or her intake ranges are within the desired range. In various embodiments, other bodily fluids (*e.g.*, urine, cerebral fluid, etc.) can be used to perform these verifications. The data of the user's actual bodily fluid makeup can allow the system to calibrate itself toe ensure that a "good" score calculated for an individual actually means that the individual is receiving adequate nutrients. For example, if a score of 100 for a particular nutrient is calculated based on the uncalibrated equations described herein, the system may use fluid measurements to determine whether the individual actually is receiving sufficient nutrients. In the event the individual is receiving too little (or too much) of a given nutrient, the fluid measurement results can be used to alter the scoring algorithms to ensure that a score of 100 actually reflects an ideal intake of a particular nutrient for a particular individual.

Further advantages of the instant disclosure will be apparent from the following detailed description and associated figures.

BRIEF DESCRIPTION OF THE FIGURES

FIG. 1 is a block diagram illustrating an example of the electrical systems of a host device usable to implement the computerized nutritional health score system disclosed herein.

FIGS. 2a, 2b, 2c, 2d, 2e, 2f, 2g, 2h, and 2i are plots of scoring functions described herein for various nutrients trackable by the disclosed system and methods.

FIG. 3 is an example of a plot of a scoring function for vitamin A based on consumed portions of beef liver according to one embodiment of the system disclosed herein.

FIG. 4 illustrates a plot comparing the nutrient score for a particular amount of calcium as calculated by the above equations in comparison to the percent of the Recommended Daily Allowance (RDA), both in comparison to relative percentiles of peoples' consumptions of calcium in the United States.

FIG. 5 is a screen shot of a plurality of nutrient health scores for consumption of an exemplary meal.

FIG. 6 is an illustration of a plurality of box plots of nutritional health scores using certain model nutrient consumption data.

FIG. 7 illustrates the individual nutrient score breakdowns for the data used to generate FIG. 6.

FIG. 8 illustrates a scatter plot of nutrient scores for the data used to generate FIG. 6.

FIG. 9 is a correlation plot of nutrient scores for the data used to generate FIG. 6.

FIG. 10 is a chart showing the evolution of the total score, sodium nutrient score, and fiber nutrient score throughout the course of a day based on data from the data used to generate FIG. 6.

FIG. 11 is a screenshot of an example dashboard showing an example of a display of a plurality of scores for a one-day diet based solely on a single meal (*i.e.*, lunch).

FIG. 12 is a screenshot of an example dashboard as in FIG. 11, with an entire day's worth of consumption illustrated.

FIG. 13 is a screenshot of an example data entry screen that enables a user to enter information about him- or herself.

FIG. 14 is a screenshot of an example screen showing an embodiment of the disclosed system in which a number of days worth of consumption can be adjusted by the user of the disclosed system.

FIGS. 15 and 16 illustrate screenshots of a plurality of nutrient health scores for a pair of individuals having different lifestyles

DETAILED DESCRIPTION

In general, the system disclosed herein calculates and displays scores indicating the impact of consumption of a nutrient. These scores are tailored to the particular individual consuming the consumable, such that the score reflects the impact of the consumption given the individual's specific needs. In addition, the score reflects whether or not endpoints have been exceeded, either by over- or under-consuming a consumable, with the score being adjusted as appropriate (*e.g.*, more drastically modified) if consumption is not within the range defined by the endpoints. In certain embodiments of the system disclosed herein, the lower endpoint (*i.e.*, the minimum amount of consumption) is determined for an individual using a combination of an EAR value and an RDA value. In certain embodiments, the upper endpoint (*i.e.*, the maximum amount that should be consumed) is determined, if it exists, using a UL value.

In certain embodiments, the disclosed system satisfies the general goal, given a particular diet, to recommend a set of new amounts of the given foods, and eventually a set of new foods, in order to improve the overall nutritional content of the diet. Since the overall score depends on the general characteristics of the individual (gender, age, weight, etc.), the recommendations to improve the overall score likewise depend on characteristics of the individual. The disclosed system and methods in these embodiments recommend either reductions or additions to the baseline meal, and further look for nutrients whose intake is above the recommended upper limit and then it calculates the minimal quantity of each food to be removed from the meal in order to keep each nutrient below its upper limit. For the additions, the system looks in the food database to determine which foods, if added to the meal, increase the global score. A set of filters can be selected to avoid specific food groups, or specific search terms. Such filtering allows for more personalized dietary advice. These recommendations, at the simplest level, try to push the baseline meal as close as possible to the healthy range for every nutrient.

In some embodiments, the system determines and stores lower and upper endpoints for nutrient intake, reflecting a minimal and maximal amount of a particular nutrient that an individual should consume over a given period of time. Generally, the scoring functionality disclosed herein is based on whether and to what extent the amount of nutrients consumed is within the endpoints, above the upper endpoint, or below the lower endpoint. The scoring described herein thus reflects an amount of over- or under- consumption by an individual.

In some embodiments, the disclosed system enables the user to indicate food items that he or she has consumed or plans to consume. For each indicated food item, a database or datastore of the disclosed system stores an indication of the nutrient content per amount of that food item. The system uses the nutritional content information, multiplied by the amount of food item consumed over time, to determine the total nutritional intake over time for that particular food item.

In various embodiments, after determining the ranges of nutrients that are optimal for a particular individual and after knowing at least one consumed or to-be-consumed food item, the system calculates one or more nutritional health scores for the individual. These nutritional health scores indicate the nutritional impact of the indicated food item. In general, these scores are calculated by determining, for each nutrient tracked by the system, whether the nutrient content of the food item falls within the optimal or healthy range for that nutrient. In general, if an individual over- or under-consumes a nutrient, the disclosed system beneficially reflects that over- or under-consumption by more drastically affecting the score for that nutrient (and thus the overall nutritional health score).

In one embodiment, a nutrient health score is calculated for each nutrient contained in a food item. The system thereafter aggregates the nutrient scores using a weighting function to indicate the relative importance of each nutrient to the overall nutritional health of the individual. In some such embodiments, the weighting function takes into account whether or not the individual has over- or under-consumed any individual nutrients; if the individual has, the disclosed system in some embodiments increases the impact of that nutrient's nutrient health score on the overall nutritional health score to reflect the fact that over- or under- consumption can have a relatively drastic impact on an individual's overall nutritional health. In an embodiment, the weighting function provides an aggregate nutritional health score on a scale of 0 to 100, where scores closer to 100 indicate greater fulfilment of the nutritional needs of the individual over a particular time period.

As further described in detail below, various embodiments the disclosed system also provide an advisory function, wherein the system suggests combinations of foods that will result in optimal nutritional health scores. For example, if a user accesses the system after breakfast and indicates the foods he or she had for breakfast, the disclosed system may calculate a nutritional health score for the breakfast foods, but may also determine what nutrients would need to be consumed over the remainder of the day for the individual to consume nutrients in the optimal ranges for all tracked nutrients that day. In this embodiment, the system uses these calculated nutrient amounts to determine combinations of food that can be consumed throughout the remainder of the day to ensure that the individual's nutritional goals are achieved as fully as possible while still consuming a number of calories within that individual's optimal caloric intake range. Thus, the system disclosed herein operates not only as a tracking system, but also as a recommendation engine to recommend consumables to help individuals reach their nutritional goals.

In embodiments of the disclosed system, the nutritional health score is calculated by determining a plurality of individual component nutrient scores, which are then aggregated into an overall nutritional health score. In various embodiments of the system and methods disclosed herein, a nutritional health score algorithm is used to calculate a nutritional health score based on a plurality of inputs, including:

- List of consumed nutrients
- Amount of consumed nutrients
- Gender
- Age
- Body Mass
- Special conditions (*e.g.*, pregnancy, lactating, etc.)

The phrase "nutrients" is used repeatedly herein. The following table contains a list of nutrients for which nutrient scores can be calculated (assuming "healthy ranges" for these nutrients are available) and from which nutritional health scores are aggregated in certain embodiments of the disclosed system:

| Amino acids | Fatty Acids | Minerals | Clinical chemistry |
|---|---|---|---|
| 1) Alanine | 1) Butyric C4:0 | 1) Li | 1) Sodium |
| 2) β-alanine | 2) Caproic C6:0 | 2) B | 2) Potassium |
| 3) Sarcosine | 3) Caprilic C8:0 | 3) Mg | 3) Chlorine |
| 4) Arginine | 4) Capric C10:0 | 4) Al | 4) Magnesium |
| 5) Monomethylarginine | 5) Undecanoic C11:0 | 5) P | 5) Calcium |
| 6) As-dimethylarginine | 6) Lauric C12:0 | 6) S | 6) Phosphorus |
| 7) Sym.-dimethylarginine | 7) Tridecanoic C13:0 | 7) K | 7) Copper |
| 8) Asparagine | 8) Myristic C14:0 | 8) Ca | 8) Iron |
| 9) Aspartic acid | 9) Pentadecanoic C15:0 | 9) Ti | 9) Ferritin |
| 10) Citrulline | 10) Palmitic C16:0 | 10) V | 10) Transferrin |
| 11) Cysteine | 11) Heptadecanoic C17:0 | 11) Cr | 11) UIBC |
| 12) Glutamic acid | 12) Stearic C18:0 | 12) Mn | 12) Holotranscobalabin |
| 13) Glutamine | 13) Arachidic C20:0 | 13) Fe | 13) Vit. B12 |
| 14) Glycine | 14) Heneicosanoic C21:0 | 14) Co | 14) TSH |
| 15) Histidine | 15) Behenic C22:0 | 15) Ni | 15) T3 free |
| 16) 1-methylhistidine | 16) Lignoceric C24:0 | 16) Cu | 16) T4 free |
| 17) 3-methylhistidine | 17) Myristoleic C14:1 | 17) Zn | 17) Albumin |
| 18) Homocysteine | 18) Pentadecenoic C15:1 | 18) As | 18) Prealbumin |
| 19) α-aminobutyric acid | 19) Palmitoleic C16:1 | 19) Se | 19) Total protein |
| 20) β-aminoisobutyric acid | 20) Heptadecenoic C17:1 | 20) Br | 20) Cholesterol |
| 21) γ-aminobutyric acid | 21) Elaidic C18:1 | 21) Rb | 21) HDL |
| 22) Isoleucine | 22) Oleic C18:1 | 22) Sr | 22) LDL |
| 23) Leucine | 23) Eicosenoic C20:1 | 23) Mo | 23) Glucose |
| 24) Lysine | 24) Erucic C22:1 | 24) Cd | 24) Triglycerides |
| 25) Methionine | 25) Nervonic C24:1 | 25) Sn | 25) 25-OH-vit D |
| 26) Ornithine | 26) Linoelaidic C18:2 | 26) I | 26) Urea |
| 27) Phenylalanine | 27) Linoleic C18:2 | 27) Cs | 28) Ceruloplasmin |
| 28) Proline | 28) Gamma-Linolenic C18:3 | 28) Hg | 29) ALAT |
| 29) Serine | 29) Alpha-Linolenic C18:3 | 29) Pb | 30) Total bilirubin |
| 30) Taurine | 30) Eicosadienoic C20:2 | | 31) Cortisol |
| 31) Threonine | 31) Eicosatrienoic C20:3 | | 32) CK (Creatine kinase) |
| 32) Tryptophan | 32) Eicosatrienoic 20:3 | | 33) Alkaline phosphatase |
| 33) Tyrosine | 33) Arachidonic C20:4 | | 34) Lipase |
| 34) Valine | 34) Docosadienoic 22:2 | | 35) hsCRP |
| 35) Hydroxyproline | 35) Eicosapentanoic C20:5 | | 36) Uric acid |
| 36) Ethanolamine | 36) Docosahexaenoic C22:6 | | 37) AST |
| | | | 38) Direct bilirubin |
| **Liposoluble vitamins** | **Hydrosoluble vitamins** | | 39) CK-MB |
| 1) All trans retinal | 1) Vit. B1 Thiamine | | 40) Creatinine |
| 2) 25-OH-vit D2 | 2) Vit. B2 Riboflavin | | 41) GGT |
| 3) 25-OH-vit D3 | 3) Vit. B5 Patothenic acid | | 42) HbA1C |
| 4) Vitamin K1 | 4) Vit. B6 Pyridoxine | | 43) LDH |
| 5) Alpha tocopherol | 5) Vit. B6 Pyridoxal P. | | 44) Folate |
| 6) Beta tocopherol | 6) Vit. B6 Pyridoxamine | | 45) PTH |
| 7) Delta tocopherol | 7) Vit. B6 Pyridoxal | | 46) acid phosphatase |
| 8) Gamma tocopherol | 8) Vit. B6 Pyridoxic acid | | 47) Apo A1 |
| 9) Alpha tocotrienol | 9) Vit. B8 Biotin | | 48) Apo B |
| 10) Gamma tocotrienol | 10) Vit. B9 Folic acid | | 49) Insulin |
| 11) Delta tocotrienol | 11) Vit. B12 Cobalamin | | 50) Thyroglobulin |
| 12) Lutein | 12) Vit. C Ascorbic acid | | 51) IgG |
| 13) Zeaxanthin | | | 52) RBP |
| 14) Beta cryptoxanthin | **Organic acids** | | 53) Vit. B3 |
| | 1) 2-ketobuyric acid | | |
| | 2) 3-methyl-2-oxobutyric acid | | |
| | 3) 3-methyl-2-oxopentanoic acid | | |
| | 4) 4-methyl-2-oxopentanoic acid | | |

In other embodiments, subsets or other sets of nutrients are used; the principles described herein with regard to the calculation of nutritional health scores applies regardless of which nutrients are accounted for in the disclosed scoring algorithm. In addition, it should be appreciated that as various nutritional bodies (*e.g.*, the Institute of Medicine) update their recommendations or add recommendations for new nutrients, the algorithms disclosed herein may be altered such as by altering the weighing of nutrients to reflect such updates.

In various embodiments, one or more of the inputs referenced above is pulled from a database of nutritional information. For example, the list of consumed nutrients may be generated in certain embodiments by allowing a user to enter a consumed item and looking up a listing of nutrients contained in that consumed item in an appropriate consumables database. In other embodiments, users enter consumed nutrients directly. In still other embodiments, a user enters a food (*e.g.*, a hamburger) and if that food is not within a database, the user also enters an amount of nutrients within that food (*e.g.*, an amount of sodium). Thereafter, future entries of the defined food (*e.g.*, a hamburger) can lookup nutrients entered at a previous time rather than requiring the user to re-enter the nutrient information.

In some embodiments, the disclosed system includes a functionality to use several measure units and serving sizes, specific to a particular food, and automatically convert between them. Therefore, a portion of one food can be entered as a given amount of grams, of kilo-calories, or according to some pre-defined serving size (cup, tablespoon, etc.), and can be converted (or normalized) to an amount of food consumed that is compatible with data stored in the database about the food.

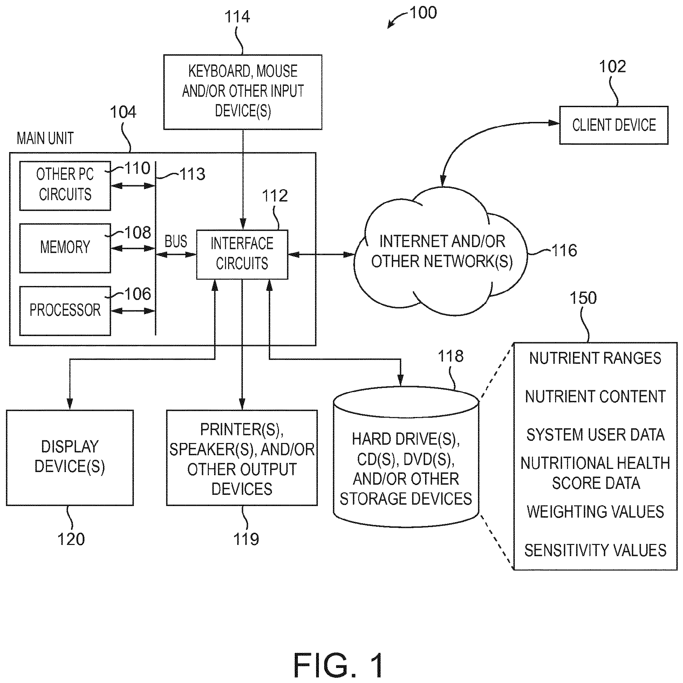

Referring now to Fig. 1, a block diagram is illustrated showing an example of the electrical systems of a host device 100 usable to implement at least portions of the computerized nutritional health score and recommendation system disclosed herein. In one embodiment, the device 100 illustrated in Fig. 1 corresponds to one or more servers and/or other computing devices that provide some or all of the following functions: (a) enabling access to the disclosed system by remote users of the system; (b) serving web page(s) that enable remote users to interface with the disclosed system; (c) storing and/or calculating underlying data, such as recommended caloric intake ranges, recommended nutrient consumption ranges, and nutrient content of foods, needed to implement the disclosed system; (d) calculating and displaying component or aggregate nutritional health scores; and/or (e) making recommendations of foods or other consumables that can be consumed to help individuals reach optimal nutritional health scores.

In the example architecture illustrated in Fig. 1, the device 100 includes a main unit 104 which preferably includes one or more processors 106 electrically coupled by an address/data bus 113 to one or more memory devices 108, other computer circuitry 110, and/or one or more interface circuits 112. The one or more processors 106 may be any suitable processor, such as a microprocessor from the INTEL PENTIUM® or INTEL CELERON® family of microprocessors. PENTIUM® and CELERON® are trademarks registered to Intel Corporation and refer to commercially available microprocessors. It should be appreciated that in other embodiments, other commercially-available or specially-designed microprocessors may be used as processor 106. In one embodiment, processor 106 is a system on a chip ("SOC") designed specifically for use in the disclosed system.

In one embodiment, device 100 further includes memory 108. Memory 108 preferably includes volatile memory and non-volatile memory. Preferably, the memory 108 stores one or more software programs that interact with the hardware of the host device 100 and with the other devices in the system as described below. In addition or alternatively, the programs stored in memory 108 may interact with one or more client devices such as client device 102 (discussed in detail below) to provide those devices with access to media content stored on the device 100. The programs stored in memory 108 may be executed by the processor 106 in any suitable manner.

The interface circuit(s) 112 may be implemented using any suitable interface standard, such as an Ethernet interface and/or a Universal Serial Bus (USB) interface. One or more input devices 114 may be connected to the interface circuit 112 for entering data and commands into the main unit 104. For example, the input device 114 may be a keyboard, mouse, touch screen, track pad, track ball, isopoint, and/or a voice recognition system. In one embodiment, wherein the device 100 is designed to be operated or interacted with only via remote devices, the device 100 may not include input devices 114. In other embodiments, input devices 114 include one or more storage devices, such as one or more flash drives, hard disk drives, solid state drives, cloud storage, or other storage devices or solutions, which provide data input to the host device 100.

One or more storage devices 118 may also be connected to the main unit 104 via the interface circuit 112. For example, a hard drive, CD drive, DVD drive, flash drive, and/or other storage devices may be connected to the main unit 104. The storage devices 118 may store any type of data used by the device 100, including data regarding preferred nutrient ranges, data regarding nutrient contents of various food items, data regarding users of the system, data regarding previously-generated nutritional health scores, data representing weighting values for calculating nutritional health scores, sensitivity values for calculating nutritional health scores, and any other appropriate data needed to implement the disclosed system, as indicated by block 150. Alternatively or in addition, storage devices 118 may be implemented as cloud-based storage, such that access to the storage 118 occurs via an internet or other network connectivity circuit such as an Ethernet circuit 112.

One or more displays 120, and/or printers, speakers, or other output devices 119 may also be connected to the main unit 104 via the interface circuit 112. The display 120 may be a liquid crystal display (LCD), a suitable projector, or any other suitable type of display. The display 120 generates visual representations of various data and functions of the host device 100 during operation of the host device 100. For example, the display 120 may be used to display information about the database of preferred nutrient ranges, a database of nutrient contents of various food items, a database of users of the system, a database of previously-generated nutritional health scores, and/or databases to enable an administrator at the device 100 to interact with the other databases described above.

In the illustrated embodiment, the users of the computerized nutritional health score and recommendation system interact with the device 100 using a suitable client device, such as client device 102. The client device 102 in various embodiments is any device that can access content provided or served by the host device 100. For example, the client device 102 may be any device that can run a suitable web browser to access a web-based interface to the host device 100. Alternatively or in addition, one or more applications or portions of applications that provide some of the functionality described herein may operate on the client device 102, in which case the client device 102 is required to interface with the host device 100 merely to access data stored in the host device 100, such as data regarding healthy nutrient ranges or nutrient content of various food items.

In one embodiment, this connection of devices (i.e., the device 100 and the client device 102) is facilitated by a network connection over the Internet and/or other networks, illustrated in Fig. 1 by cloud 116. The network connection may be any suitable network connection, such as an Ethernet connection, a digital subscriber line (DSL), a Wi-Fi connection, a cellular data network connection, a telephone line-based connection, a connection over coaxial cable, or another suitable network connection.

In one embodiment, host device 100 is a device that provides cloud-based services, such as cloud-based authentication and access control, storage, streaming, and feedback provision. In this embodiment, the specific hardware details of host device 100 are not important to the implementer of the disclosed system-instead, in such an embodiment, the implementer of the disclosed system utilizes one or more Application Programmer Interfaces (APIs) to interact with host device 100 in a convenient way, such as to enter information about the user's demographics to help determine healthy nutritional ranges, to enter information about consumed foods, and other interactions described in more detail below.

Access to device 100 and/or client device 102 may be controlled by appropriate security software or security measures. An individual user's access can be defined by the device 100 and limited to certain data and/or actions, such as inputting consumed food or viewing calculated scores, according to the individual's identity. Other users of either host device 100 or client device 102 may be allowed to alter other data, such as weighting, sensitivity, or healthy range values, depending on those users' identities. Accordingly, users of the system may be required to register with the device 100 before accessing the content provided by the disclosed system.

In a preferred embodiment, each client device 102 has a similar structural or architectural makeup to that described above with respect to the device 100. That is, each client device 102 in one embodiment includes a display device, at least one input device, at least one memory device, at least one storage device, at least one processor, and at least one network interface device. It should be appreciated that by including such components, which are common to well-known desktop, laptop, or mobile computer systems (including smart phones, tablet computers, and the like), client device 102 facilitates interaction among and between each other by users of the respective systems.

In various embodiments, devices 100 and/or 102 as illustrated in Fig. 1 may in fact be implemented as a plurality of different devices. For example, the device 100 may in actuality be implemented as a plurality of server devices operating together to implement the media content access system described herein. In various embodiments, one or more additional devices, not shown in Fig. 1, interact with the device 100 to enable or facilitate access to the system disclosed herein. For example, in one embodiment the host device 100 communicates via network 116 with one or more public, private, or proprietary repositories of information, such as public, private, or proprietary repositories of nutritional information, nutrient content information, healthy range information, environmental impact information, or the like.

In one embodiment, the disclosed system does not include a client device 102. In this embodiment, the functionality described herein is provided on host device 100, and the user of the system interacts directly with host device 100 using input devices 114, display device 120, and output devices 119. In this embodiment, the host device 100 provides some or all of the functionality described herein as being user-facing functionality.

The system of Fig. 1 is configured to calculate nutrient health scores and overall nutritional health scores by using the illustrated hardware to perform algorithms implementing the Equations described in more detail below for calculating scores. Those of skill in the art will understand that this functionality is not general-purpose computer functionality, but requires the computer to be specially programmed with instructions to calculate results of the Equations listed below, and to otherwise perform the various algorithms described in various forms herein.

In various embodiments, the system disclosed herein is arranged as a plurality of modules, wherein each module performs a particular function or set of functions. The modules in these embodiments could be software modules executed by a general purpose processor, software modules executed by a special purpose processor, firmware modules executing on an appropriate, special-purpose hardware device, or hardware modules (such as application specific integrated circuits ("ASICs")) that perform the functions recited herein entirely with circuitry. In embodiments where specialized hardware is used to perform some or all of the functionality described herein, the disclosed system may use one or more registers or other data input pins to control settings or adjust the functionality of such specialized hardware. For example, a hardware module may be used that is programmed to analyze nutrient health scores based on a piecewise continuous function that is increasing in a first segment, flat in a second segment, and decreasing in a third segment. In this example, the hardware may be programmed to evaluate the function, and one or more inputs to the hardware may be configured to receive inputs of, for example, the input value at which the first segment meets the second segment, the input value at which the second segment meets the third segment, and parameters to indicate the rate at which the third segment is decreasing (*e.g.*, a slope or a function defining the shape of the third segment). In still other embodiments, where the modules to perform various functionality described herein are software modules executable by hardware, the modules may take the form of apps or subsets of apps that may be designed to run on a processor executing a particular, predefined operating system environment.

In another embodiment, one or more devices carried by the user provide real-time information to the system when the user is in a food purchasing establishment such as a grocery store or a restaurant. Devices such as RFID readers, NFC readers, wearable camera devices, and mobile phones could receive or determine (such as by scanning RFID tags, reading bar codes, or determining the physical location of a user) foods that are available to a user at a particular grocery store or restaurant. The disclosed system then makes recommendations taking into account what foods could be immediately purchased or consumed by the user. In one such embodiment, when a user sits down at a restaurant, the disclosed system may push information to the user's mobile phone recommending that the user select certain items from the menu to optimize the user's nutritional health score for a given time period. In still other embodiments, a voice recognition feature recognizes inputs provided vocally by a user. In one such embodiment, the voice recognition system listens as a user orders at a restaurant; in other embodiments, the voice recognition system enables the user to speak directly the items he or she has consumed or will consume.

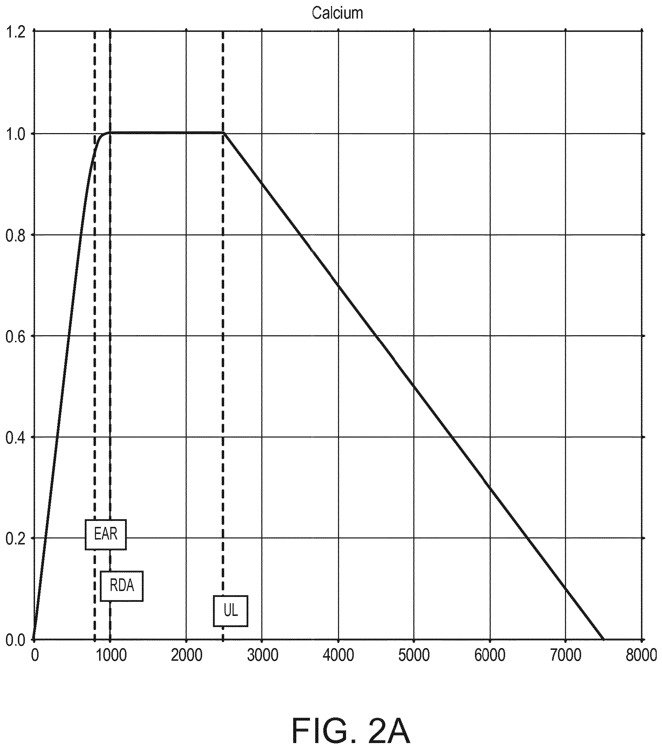

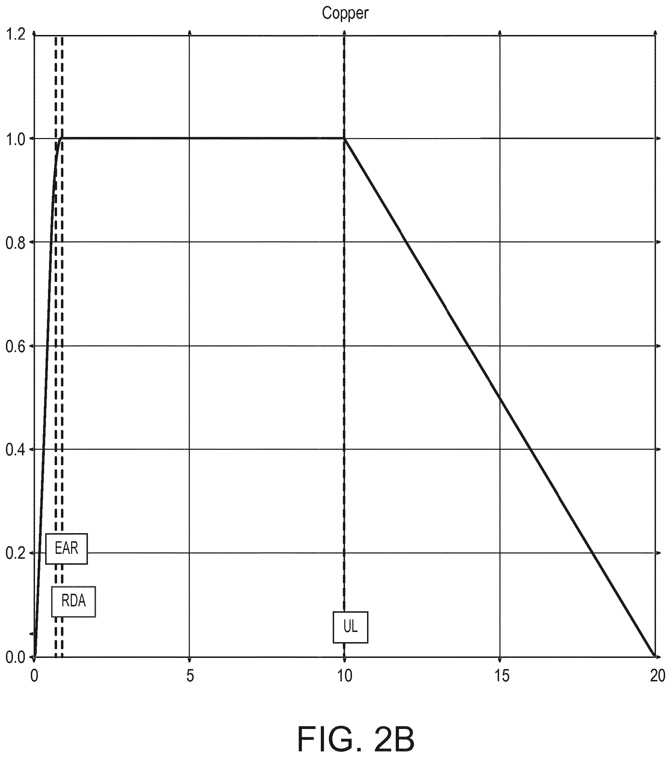

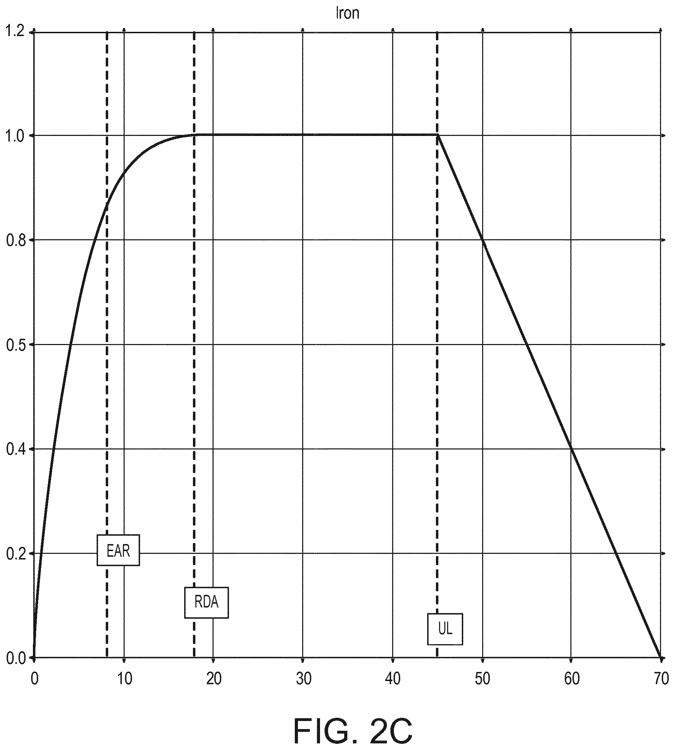

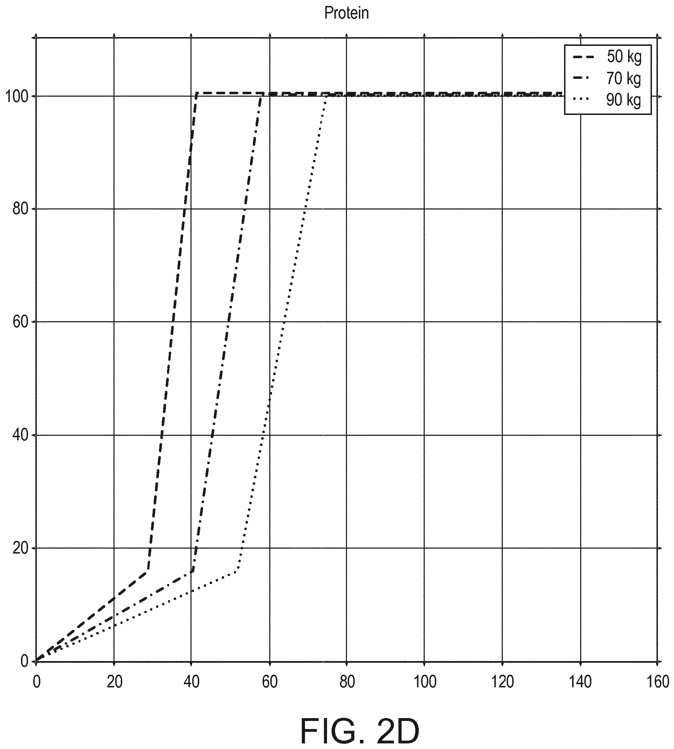

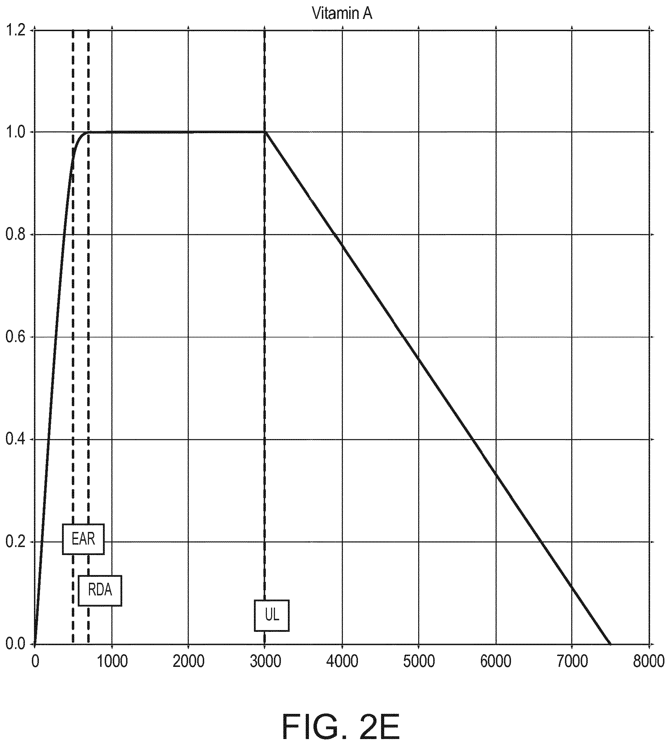

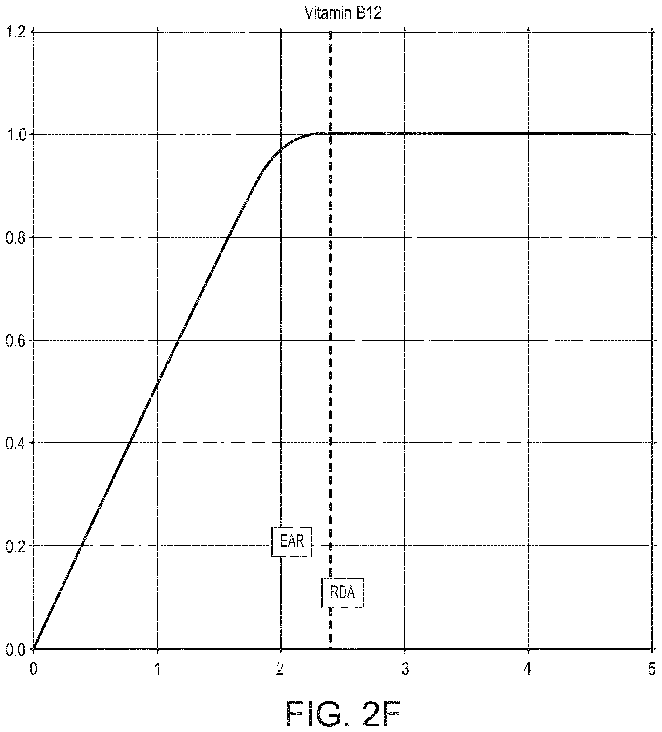

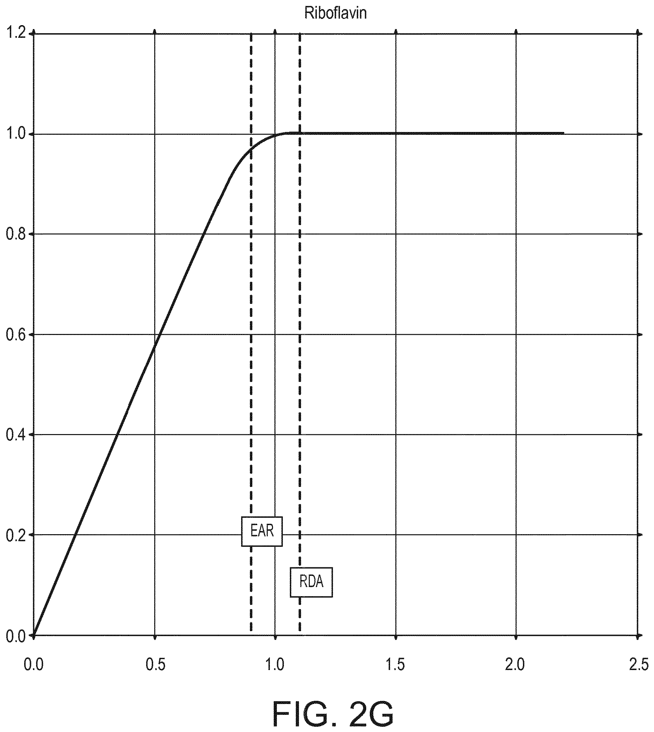

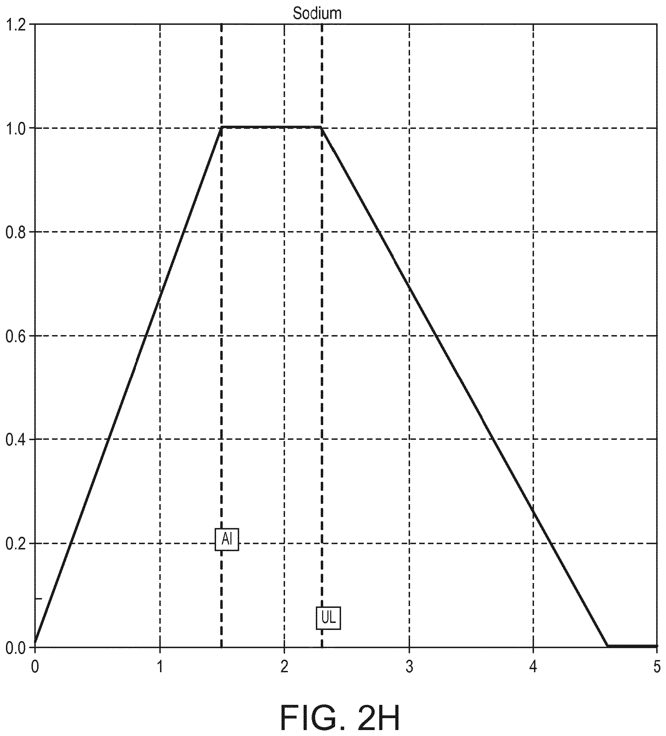

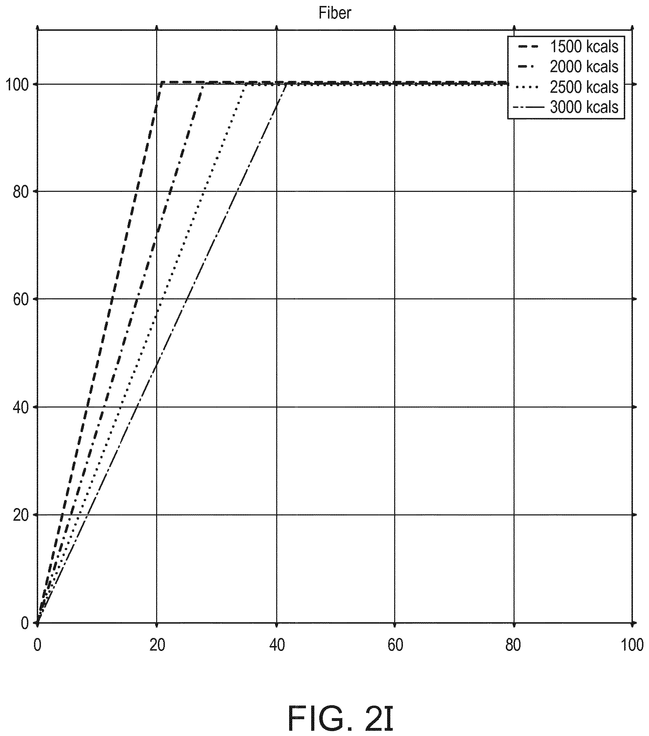

In one embodiment, the system disclosed herein calculates a score between 0.0 and 1.0 for each nutrient. In this embodiment, the intake range (and thus the scoring function) are divided into three different regions: (1) intakes of the nutrient between 0.0 and the lower consumption limit, which may be based on a combination of the EAR and RDA; (2) intakes between the lower consumption limit and the upper limit, which is equivalent to UL; and (3) intakes above the upper consumption limit. In this embodiment, intakes in the first range indicate a potential deficiency; the nearer the intake amount is to the RDA, the less likely the intake is actually insufficient. The second range may be described as the "hemostasis region," wherein the score approaches and/or is equal to the maximum value for that nutrient (*e.g.*, 1.0). The third range reflects over-consumption, and is an intake range whereby chronic intakes in the range are generally not recommended. In the described embodiment, scores for a particular nutrient within the third range decrease until they reach a minimum score (*e.g.*, 0). The specific equations to calculate these scoring functions are detailed below. In addition, Figs. 2a, 2b, 2c, 2d, 2e, 2f, 2g, 2h, and 2i illustrate plots of some of the disclosed scoring functions using exemplary values for EAR, RDA, UL, and AI. Where appropriate, those figures will be referenced in describing the various Equations in the following discussion.

It should be appreciated that many factors can impact the rapidity of the decrease, including: (a) the uncertainty in establishing the UL; (b) the gravity or severity of possible adverse effects from exceeding the UL; and (c) factors specific to the consuming individual (*e.g.*, age). It should be further appreciated that several additional factors could impact both the rapidity with which the function approaches a minimum, and the shape of the curve approaching the minimum.

The algorithms described below, in various embodiments, use a smoothing process or procedure to reflect the distributional properties of dietary reference intakes. In particular, when enough information is available for a particular nutrient, an EAR and an RDA are available based on the assumption of normally distributed requirements for a given population (*e.g.*, for a given gender/age group). In an embodiment, the relationship between these two quantities can be described according to the following equation:

$$(2) \qquad RDA = EAR + 2\sigma$$

In Equation 1, "$\sigma$" is the standard deviation. Accordingly, the minimal requirements in this embodiment follow a normal distribution with the mean $\mu = EAR$ and standard deviation $\sigma = (RDA - EAR) / 1.96$:

$$(2) \qquad X \sim N(\mu, \sigma) = N\left(EAR, \frac{RDA - EAR}{1.96}\right)$$

The algorithm operating on the system disclosed herein, in some embodiments, is a piecewise linear function defined as $f(x) = x / RDA$; in these embodiments, a Gaussian smoothing kernel is thereafter used as shown in Equation 3:

$$(3) \qquad score(x) = S(x) = \int_0^x \frac{e^{-\frac{(t-EAR)^2}{2\sigma^2}}}{\sigma\sqrt{2\pi}} dt + \int_x^{RDA} \frac{x}{t} \frac{e^{-\frac{(t-EAR)^2}{2\sigma^2}}}{\sigma\sqrt{2\pi}} dt$$

The above function includes several properties unique to the algorithm (and system) disclosed herein, including:

- *S* is a smooth function on the interval [0, RDA]
- *S* is a concave function
- $S(0) = 0$ and $S(RDA) = 1.0$
- $S'(RDA) = 0$, meaning the tangent at $x = RDA$ is horizontal

In some embodiments, the above function is re-scaled so that its value is at 1.0 when x = RDA; it should be appreciated that that this re-scaling does not meaningfully affect the various properties of this function. The scoring functions for Calcium, Copper, Iron, and Vitamin A, illustrated by Figs. 2a, 2b, 2c, and 2e, are examples of these types of functions.

It should be appreciated that the estimation of the requirements for protein intake represents a special case. First, the required protein intake depends on the body mass of the individual, and is generally represented in terms of g/kg/day. Second, the distribution of protein requirements is estimated to be log-normal, where the relationship between RDA and EAR can be approximated by the following Equation 4:

$$(4) \qquad RDA = 1.24 * EAR$$

Thus the intake estimate for adequate protein intake for a percentile P of a healthy population is given by the following Equation 5:

$$(5) \qquad \exp[0.12 * z(P) - 0.425]$$

In Equation 5, $z(P)$ is the value of the standardized normal distribution at the percentile P. The RDA is estimated as the halfway point between the 16th and the 84th percentiles for the log-normal distribution. These percentiles can also be used as reference points for a piecewise linear interpolation to calculate the nutrient score for an amount $x$ of protein according to the following Equation 6:

$$(6) \qquad score(x) = \frac{84}{RDA - 16^{th} percentile}\left(x - 16^{th} percentile\right) + 16$$

The RDA for total protein can be estimated as 0.8 g/kg/d for men and women ages 19-50. Notably, this RDA value is dependent upon the weight of the individual - individuals having different weights have different RDAs of protein. By virtue of the fact that the RDA value for protein varies with the weight of an individual, Fig. 2d illustrates a plurality of scoring functions for protein for individuals having different weights (and thus slightly different scores for various amounts of consumed protein).

It should be appreciated that in some cases, EAR and RDA for particular nutrients may not be known. For example, some important nutrients, like sodium, potassium, and vitamin K may not have known EAR and RDA values. For these types of nutrients, a recommended minimal intake (referred to as an Adequate Intake or AI) may be established. With regard to AI, the Institute of Medicine has noted that if an individual's usual intake equals or exceeds the AI, it can be concluded that the diet is almost certainly adequate. If, however, their intake falls below the AI, no quantitative (or qualitative) estimate can be made of the probability of nutrient inadequacy. Thus, while the AI is believed to represent an amount of a nutrient sufficient to satisfy the needs of healthy individuals, lack of sufficiently accurate data means it is difficult or impossible to estimate the percentage of individuals covered by this AI value.

According to embodiments of the system and methods disclosed herein, the nutrient scoring algorithm for nutrients whose intake levels are defined in terms of "adequate" intakes has a lower portion of the curve defined by the following simplified Equation 7:

$$(7) \qquad S(x) = \min\left(1, \frac{x}{AI}\right)$$

In this simplified equation, x is defined as the intake (in the appropriate units) between 0 and the UL value for that nutrient.

In various embodiments, the disclosed system also implements a special algorithm for the nutrient sodium. With regard to sodium, the Institute of Medicine has defined an AI and an UL, but has not defined an EAR/RDA pair for this element due to the lack of lack of adequate data when the Institute of Medicine reports were written. Sodium is the principal cation of extra-cellular fluid and functions as the osmotic determinant in regulating extra-cellular fluid volume. Sodium is also an important determinant of the membrane potential of cells and regulates the active transport of molecules across cell membranes. Therefore it is important to guarantee that sodium intake is sufficient to maintain an adequate concentration in the extra-cellular fluid. On the other hand, a high intake of sodium has well known negative effects on hypertension, so it is important to include an upper limit as well.

Given the importance of this element from the physiological point of view, and from the point of view of public health, the system disclosed herein nonetheless includes sodium in the overall nutritional health score by calculating a nutrient score for sodium and accounting for that score in the aggregate nutritional health score. In various embodiments of the disclosed system, the algorithm determines the standard for a minimum score of 0 for sodium by using information about the usual intake from food and beverages in the US population. Using this approach, the following Equation 8 can be used to determine the nutrient health score for sodium:

$$(8) \qquad S(x) = 100 \cdot \min\left(\frac{x}{1.5}, 1, -(x-2.3)/2.3+1\right)$$

In this equation, the value of 1.5 represents the AI recommended for the population (here, a female of ages 31 - 50 without pregnancy or lactation), the value of 2.3 represents the tolerable upper level, and the line -(x-2.3)/2.3+1 crosses the horizontal axis at about 4.6 grams, corresponding to the 95th percentile of usual intake from food and beverage in the United States. Accordingly, with regard to sodium, Equation 8 reflects the fact that too much sodium intake can actually be harmful, and can have a negative impact on the overall nutritional health score (as opposed to a value of 0, which would have no positive impact but also no negative impact. Fig. 2h illustrates an example plot of a plurality of nutrient health scores for sodium.

In various embodiments of the disclosed system, the intake of dietary fibers is evaluated with regard to the overall energy intake. In general, the recommendation for dietary fiber AI is 14 grams per 1000 kcals for adults 19 years old and greater. The Institute of Medicine has not established an upper limit for intake of dietary fibers. Accordingly, embodiments of the system disclosed herein evaluate a nutrient score for dietary fiber by first determining the AI of dietary fiber by multiplying total energy intake E by 14 and dividing by 1000. With the calculated AI for dietary fiber, the disclosed system evaluates the score according to the following Equation 9:

$$(9) \qquad S(x) = 100 \cdot \min\left(\frac{x}{AI}, 1\right)$$

Accordingly, embodiments of the disclosed system improve known scoring techniques by, among other things, calculating a score for protein, sodium, and/or dietary fiber differently from how scores for other nutrient health scores are calculated. Fig. 2i illustrates an exemplary plot of nutrient health scores for fiber based on different amounts of energy intake.

It should be appreciated that for certain nutrients, like Vitamin B12 and Riboflavin, the Institute of Medicine has not found sufficient scientific evidence to set a Tolerable Upper Intake Level (UL). In many cases, it should be appreciated that this reflects a relatively low (or zero) known toxicity for the particular nutrients. Accordingly, for these nutrients, the score curve is flat for any intake above the RDA value. This shape can be seen, for example, in Fig. 2f (regarding Vitamin B12).

In some embodiments of the system and methods disclosed herein, intakes of particular nutrients are assessed or evaluated with respect to a recommended maximal intake, referred to variously as UL. In general, the estimate of the UL is based on a dose-response assessment, and is built upon three toxicological concepts commonly used in assessing the risk of exposures to chemical substances:

- LOAEL (Lowest Observed Adverse Effect Level): reflects the lowest continuing intake at which an adverse effect has been identified.
- NOAEL (No Observed Adverse Effect Level): reflects the highest continuing intake of a nutrient at which no adverse effects have been observed in the individuals or groups studied. When the available data are not sufficient to reveal a particular NOAEL, it is necessary to rely on a LOAEL.
- UF (Uncertainty Factor): factors applied when determining a NOAEL, and if necessary when determining the LOAEL, to attempt to address both gaps in data and incomplete knowledge regarding the inferences required. The ULs are generally referred to as NOAEL/UF, or as LOAEL/UF in the case where no NOAEL is available.

It should be appreciated that UF ≥ 1 for all nutrients, reflecting the fact that the UL will generally be smaller than or equal to the NOAEL or the LOAEL. Typical values for UF are between 1 and 5, but can also be higher in special cases (*e.g.*, UF = 36 for α=Tocopherol).

To illustrate the importance of setting appropriate tolerances, consider the following two examples, where upper limits are reached with normal consumption of ordinary foods.

In one example, 28 grams of Brazil nuts (1 oz., approximately six nuts) contain 537 micro-grams of Selenium, which is well above the conventionally accepted upper limit (400 micro-grams per day). However, the NOAEL is 800 micro-grams per day, so the score determined by the disclosed system would not be heavily penalized. In an example calculation, 28 grams of Brazil nuts give a relatively good score of 0.83 for Selenium. However, given low tolerance to Selenium, the disclosed system would calculate a score of 0 when 63 grams of Brazil nuts (more than 13 nuts), which contain 1200 micro-grams of Selenium, are consumed.

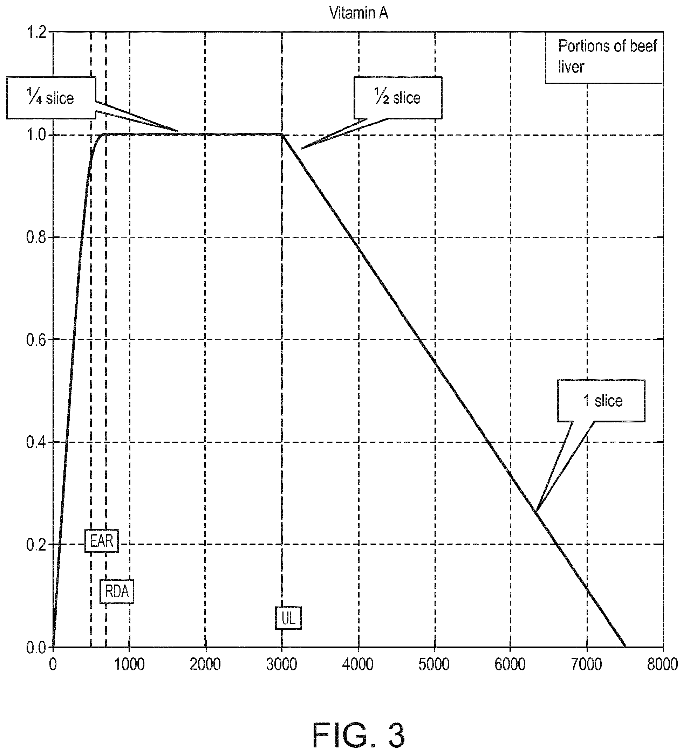

A second example, involving calculating nutrient health scores for Vitamin A from eating braised beef liver, is illustrated with regard to Fig. 3. Specifically, 'Beef liver, braised', an entry in the USDA's Food and Nutrient Database for Dietary Studies (FNDDS), contains 1779 micro-grams of vitamin A per 19 grams (1 oz.). A typical slice of beef liver is about 69 grams, meaning that a typical slice contains 6367 micro-grams of vitamin A. The RDA and UL for a female of age 31-50 are, respectively, 700 and 3000 micro-grams per day for preformed vitamin A. However, because vitamin A is fat soluble, the body stores excess amounts, primarily in the liver, and these levels can accumulate to a more harmful level. While an occasional intake of vitamin A between the RDA and the NOAEL is not considered a serious risk of toxicity for vitamin A, excess preformed vitamin A can have significant toxicity (known as hypervitaminosis A). Chronic intakes of excess vitamin A can lead to increased intra-cranial pressure, dizziness, nausea, headaches, and other symptoms. The resulting liver damage is not always reversible. The instant system takes this fact into account in calculating its score for vitamin A by setting a low tolerance for intakes above the upper limit. In addition, the instant system can warn the user about the potential risks associated with chronic high intakes of the particular nutrient. In some embodiments, the disclosed system periodically analyzes pat intakes to determine whether a user is frequently consuming high amounts of vitamin A; in these embodiments, the system flags such frequent intake as a potential overall diet problem. In other embodiments, the system is configured to score a diet over the course of a longer period of time than a day (*e.g.*, over the course of a month). In these embodiments, while the system may not detect a month-long over-consumption of vitamin A, it may flag that the user has drastically exceeded the upper limit for vitamin A on ten different days in the month. In these embodiments, the system thus displays a warning to the user to indicate repeated (albeit not every day) overconsumption of vitamin A.

It is important to note that, for some vitamins and minerals, toxicity from food intake is unlikely; however, food intake added to supplemental intake can reach very high levels, whose adverse effects can represent a serious danger and provoke, in some cases, permanent damage. In one embodiment, the instant system takes this fact into account by allowing the user to enter the supplements he or she may be consuming. Thereafter, the system determines product compositions for the supplements from an appropriate data store (*e.g.*, a comprehensive supplements database). The system then factors these supplements into an individual's overall diet and scores the overall diet accordingly.

Embodiments of the system disclosed herein take account of NOAEL and LOAEL above the UL value. In one such embodiment, the following Equation 10 reflects the calculation of a nutrient score for intake amounts x above the UL:

$$(10) \qquad S(x) = \max\left(0, 100 - k \cdot \frac{100}{NOAEL} \cdot (x - UL)\right)$$

In this Equation 10, *k* is a free parameter that can be adjusted according to particular needs. In general, in this embodiment, the higher the value of *k*, the faster the score will decrease to 0 above the UL value. In some embodiments, the disclosed system does not allow for negative values of the score S; in such embodiments, the score S is simply set to 0 for very high intakes of particular nutrients. That is, when the value of Equation 10 above takes a negative value, the algorithm automatically sets the nutrient score for that particular nutrient to zero.

In various embodiments, when a LOAEL, instead of a NOAEL, is specified for a particular nutrient, the algorithm executed by the disclosed system performs the following Equation 11 for amounts of nutrient intake above the UL:

$$(11) \qquad S(x) = \max\left(0, 100 - \frac{100}{UL - LOAEL} \cdot (x - UL)\right)$$

It should be noted that in one embodiment, the Equation 11 is only applicable if UL $\neq$ LOAEL. In addition, in various embodiments, the disclosed system does not permit negative values of S for Equation 11; in this case, the function merely sets a value of zero when the calculated score is less than zero.

In an embodiment, a separate score for the main groups of macro-nutrients is calculated. Energy for the metabolic and physiological functions of humans is derived from the chemical energy bound in the 4 groups of macro-nutrients: carbohydrates, fats, proteins, and ethanol. These groupings are generated from the fact that energy for the metabolic and physiological functions of humans is derived from the chemical energy bound in the 4 groups of macro-nutrients. Fats and carbohydrates are the main dietary sources of energy. For any given diet consumed by an individual, the sum of the contribution to energy intake as a percentage of total intake for carbohydrate, fat, protein, and alcohol must equal 100 percent. This is summarized in the following Equation 12:

$$(12) \qquad E = P + C + F + \Delta$$

In this equation, E is energy, P is the contribution to energy from proteins, C is the contribution to energy from carbohydrates, F is the contribution to energy from fats, and $\Delta$ is the contribution to energy from ethanol.

In general, there is no known dietary reference intake for energy. Body weight provides each individual with a readily monitored indicator of the adequacy or inadequacy of habitual energy intake. Large daily deviations from energy balance are thus readily tolerated, and accommodated primarily by gains or losses of body fat. There exist various equations to predict the energy requirement; the Institute of Medicine Equation, for example, estimates an individual's energy requirement based on gender, age, weight, height and physical activity level.

While this rough technique is sometimes convenient to roughly estimate the energy needs of an individual, inter-individual energy requirement variations are high, with coefficients of variation for intra-individual variability in daily energy intake averaging approximately 23%. Given these considerations, the instant disclosure has recognized that it is inappropriate to calculate a score for the energy based only on estimates made at a population level. Therefore, in various embodiments of the disclosed system, energy intake is generally not included in the calculation of the score. However, a range of acceptable values for energy can be used as a constraint when optimizing a diet, as will be shown in more detail below.

Analyzing energy intake, for a given energy intake, increases in the proportion of one macro-nutrient necessarily involves a decrease in the proportion of the other macro-nutrients. Typically, fats and carbohydrate are inversely related to each other, such that an increase in consumption of fats reduces the required consumption of carbohydrates. There is a growing body of evidence that a major imbalance in the relative proportions of macro-nutrients can increase risk of chronic disease and may adversely affect micro-nutrient intake. The Institute of Medicine recommends that the protein should contribute between 10% and 35% of the energy intake, with 45%-65% coming from carbohydrate and 20%-35% coming from fat. It is important to note that these relationships are accompanied by some degree of inter-dependency: if for example energy from protein accounts for 35% and energy from carbohydrates accounts for 65%, the energy from fats is necessarily 0%. Moreover, carbohydrate and protein must also satisfy a recommended dietary allowance, defined as a minimum amount of grams to be consumed on a daily basis, for reasons independent of energy generation. Ethanol should not be a primary source of energy, so the instant disclosure in various embodiments imposes a "penalty" on the overall nutritional health score to account for energy from alcoholic drinks.

Accordingly, in one embodiment of the system disclosed herein, the disclosed scoring algorithm is configured to calculate a separate score for the four macro-nutrients discussed above. The following equations can be used by this embodiment of the system disclosed herein to determine a nutrient score based on energy from the four various sources of energy. In this discussion, $x_P$ is used to denote the fraction of energy derived from protein, $x_F$ is used to denote the fraction of energy derived from fats, and $x_C$ is used to denote the fraction of energy derived from carbohydrates. Because total energy is the sum of energy from protein, fats, carbohydrates, and ethanol, the sum of $x_P + x_F + x_C$ must be less than or equal to 1. In the event the sum is less than 1, the difference between the sum and 1 is the fraction of energy that comes from ethanol.

In embodiments of the system disclosed herein, the algorithms impose a constraint that 20%-35% of energy should come from fat. This constraint is achieved with the following Equation 13:

$$(13) \qquad f_F(x_F) = \min\left( \frac{x_F}{0.2}, 1, -\frac{x_F - 0.35}{0.65} + 1 \right)$$

In embodiments of the system disclosed herein, the algorithms impose a constraint that 10%-35% of energy should come from protein. This constraint is achieved with the following Equation 14:

$$(14) \qquad f_P(x_P) = \min\left( \frac{x_P}{0.1}, 1, -\frac{x_P - 0.35}{0.65} + 1 \right)$$

In embodiments of the system disclosed herein, the algorithms impose a constraint that 45%-65% of energy should come from carbohydrates. This constraint is achieved with the following Equation 15:

$$(15) \qquad f_C(x_C) = \min\left( \frac{x_C}{0.45}, 1, -\frac{x_C - 0.65}{0.35} + 1 \right)$$

In an embodiment of the disclosed system, the overall macro-nutrient score is calculated as the minimum of either the individual score for the three sources of energy or for the sum of the three sources of energy, and can be represented by the following Equation 16:

$$(16) \qquad score(\mathrm{macro-nutrients}) = \min\left(f_P(x_P), f_C(x_C), f_F(x_F), x_P + x_C + x_F\right)$$

Equations 13, 14, 15, and 16 all result in results between 0 and 1, and if desired the disclosed system can scale the result of Equation 16 (the overall score for macro nutrients) to be in the range of 0-100 by multiplying by 100.

To illustrate the overall score from macro-nutrients, the following examples are presented:

Example 1 - If the energy comes entirely from carbohydrate, protein or fat, then the macro-nutrients score is 0.
Example 2 - If xF = xC = xP = 0, then the macro-nutrients score is 0.
Example 3 - If xF = 0.35; xC = 0.55; xP = 0.1, then the macro-nutrients score is 100.

In various embodiments, as noted above, the disclosed system aggregates several individual nutrient scores into an aggregated, single, global nutritional health score. In an example involving n individual nutrient scores $s_1,...,s_n$, the nutritional health score represents an overall measure of the nutritional adequacy of an individual's diet expressed as S = $M(s_1,...,s_n)$. In embodiments of the system disclosed herein, the following four general requirements apply for the aggregated score S:

1. Value preservation, such that if each individual score is a particular value, the overall value S is equal to the same particular value. This can be expressed as $M(a,a,...,a) = a$.
2. S should be sensitive to extreme individual nutrient scores, such that if a single nutrient has very bad score, the overall nutritional health score should be low and the quality of the diet should be considered low.
3. The aggregate score should be between the lowest individual nutrient score and the highest individual nutrient score. This can be expressed as $\min(s_1,...,s_n) \leq M(s_1,...,s_n) \leq \max(s_1,...,s_n)$.
4. If one of the inputs $s_1,...,s_n$ is 0, then $M(s_1,...,s_n)$ should also be equal to 0.

The equation that satisfies the above reflected requirements, which is applied by various embodiments of the system disclosed herein, is reflected in Equation 17 below:

$$(17) \qquad M(s_1,...,s_n) = \left(\prod_{i=1}^{n} s_i\right)^{1/n} = \exp\left(\frac{\sum_i \ln s_i}{n}\right)$$

It should be appreciated that this Equation 17 represents a curve that is concave, which is important for the optimization theory. In further embodiments of the system and method disclosed herein, the above Equation 17 can be further generalized by introducing specific weight values for each nutrient, which can be stored in the system and used to reflect specific characteristics of the users, environment, or food consumed, as is appropriate. These weighting values are described elsewhere herein.

In various embodiments, the system disclosed herein is configured to determine a nutrient score for fatty acids. In some such embodiments, the algorithm is the same algorithm used in the Healthy Eating Index: the score reflects the ratio of unsaturated to saturated fatty acids and has a maximum value (*e.g.*, 1) if the ratio is at least 2.5 and minimum value (*e.g.*, 0) if the ratio is below 1.2.

In an embodiment, the disclosed system compensates for the bias that is possibly introduced by missing values in the nutritional breakdown. That is, the algorithm disclosed herein is capable of calculating a usable nutritional health score even if certain nutrient scores are missing due to lack of data about nutrient intake. If a given nutrient has some missing values in the nutrient breakdown, the disclosed system in certain embodiments compensates for the missing information by re-weighting the mean. If $\alpha_i$ is the fraction of missing values for nutrient i in the diet, the disclosed system stores an indication of the weight $w_i = 1 - \alpha_i$ so that the overall score calculation is reflected in the following Equation 18:

$$(18) \qquad M(s_1,...,s_n) = \exp\left(\frac{\sum_i \ln w_i s_i}{\sum_i s_i}\right)$$

It should be appreciated that in one extreme case, $\alpha_i = 0$ (meaning there are no missing values). In this case, $w_i$

(the weight given to each score $s_i$) is 1, reflecting that each individual nutrient score is legitimately calculated. In the other extreme case, $\alpha_i = 1$ (meaning that all values are missing). In this case, $w_i$ (the weight given to each score $s_i$) is 0, reflecting the fact that no scores are legitimate.

In one embodiment, the system and methods disclosed herein are configured to score a diet over the course of several days, despite the fact that scoring is by default configured to be based on daily intake recommendations. The disclosed system is capable of handling diets over the course of several days in one of two ways.

First, the system in some embodiments is configured to calculate the average of n scores over n days and return the average score as the score for those n days.

Second, the system in some embodiments is configured to multiply all the dietary reference intakes by n and score the diet after adding all the meals together.

The calculations resulting from these two methods actually produce the same n-day score if all the nutrient daily intakes are below the minimal recommended intake (RDA or AI). However, as a practical matter, at least some of the daily intake amounts will likely fall between the RDA and the UL, and some may be above the UL. In this case, given the non-linearity of the score function, the results from methods 1 and 2 will be different. In some embodiments, the second method will produce a larger score number than the first method because adding together the amounts of different days allows for the compensation of one day under the RDA with another day over the UL, for example. Therefore, the interpretation of the n-day score will be different in the two cases. In fact, it is theoretically possible to have a low score in every single day, and still have an overall high score with the method (2).

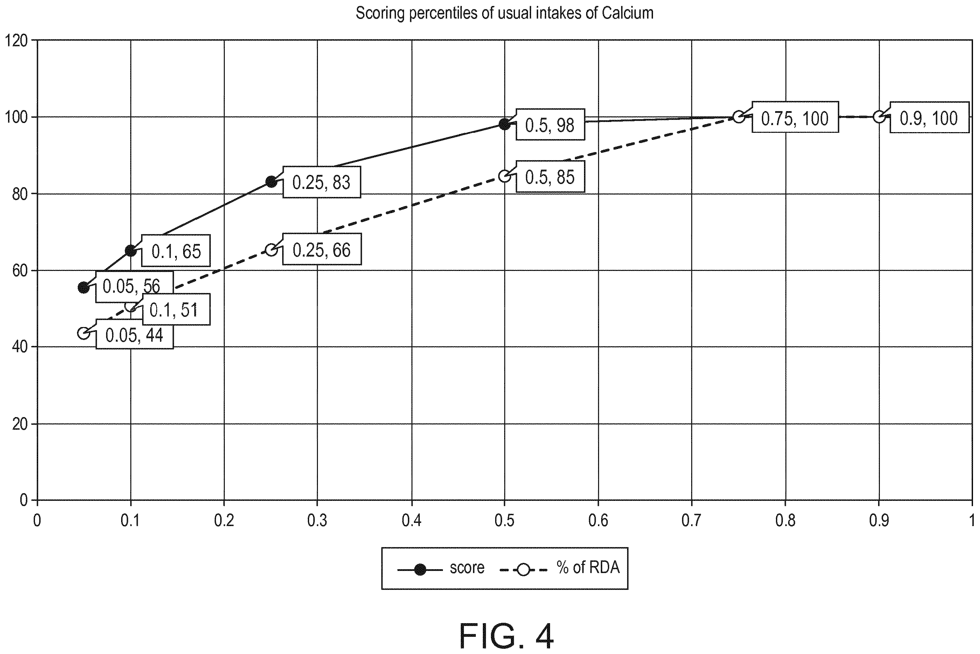

To further illustrate the scoring described herein, Fig. 4 shows a plot comparing the nutrient score for a particular amount of calcium as calculated by the above equations in comparison to the percent of the Recommended Daily Allowance (RDA), both in comparison to relative percentiles of peoples' consumptions of calcium in the United States. As can be seen by the chart, up to the seventh-fifth percentile, scores are higher than the percent of RDA intake for calcium for the screened population. Above the seventy-fifth percentile, nutrient health scores were 100 and the scored individuals consumed 100% of the RDA of calcium. This indicates that the choice of the RDA as a reference value for an individual can lead to a significant overestimate of the actual individual requirements. The USDA estimates the prevalence of apparently inadequate intakes as the proportion of individuals with usual intakes below the EAR (as opposed to below the RDA). This prevalence is usually considerably lower than the proportion of individuals whose usual intake is below the RDA. Accordingly, as Fig. 4 makes clear, achievement of RDA is, for a majority of people, unnecessary to obtain inadequate intake.

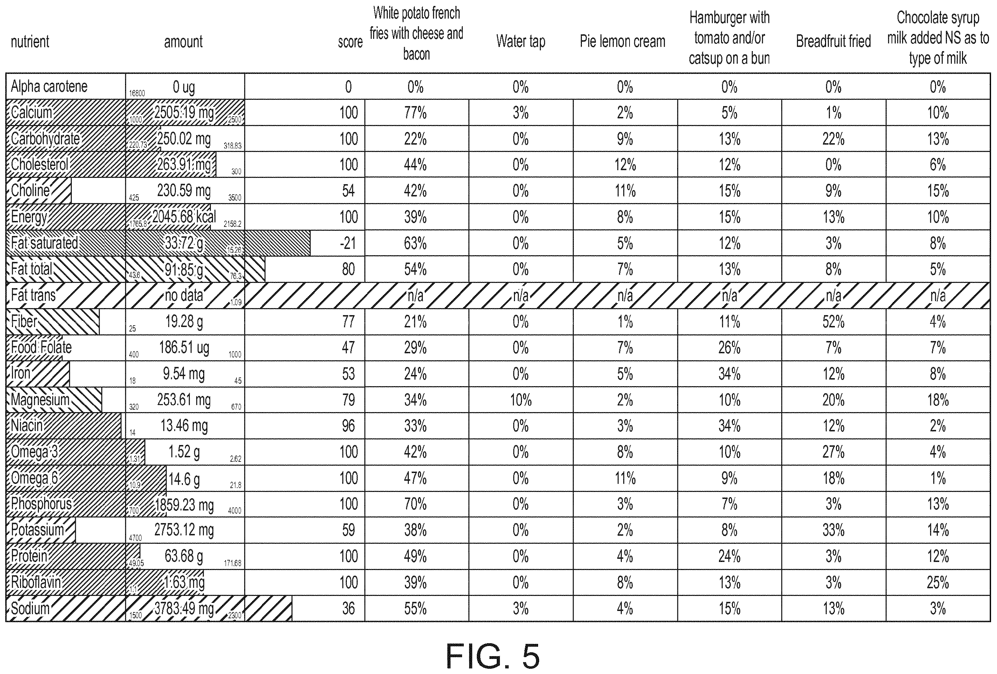

Fig. 5 is an exemplary screen shot of a screen displayed by the system disclosed herein, representing nutrient scores for a plurality of nutrients consumed during the course of a single meal. In the screen shot of Fig. 5, the meal includes French fries with cheese and bacon, tap water, lemon cream pie, a hamburger with tomato, catsup, and a bun, breadfruit (fried), and chocolate milk. The left-most column indicates the individual nutrient health scores for the different nutrients listed in the left-most column, alphabetically from alpha carotene to sodium. In this embodiment, several nutrient scores are 100 (*e.g.*, calcium), and at least one score (saturated fat) is negative, indicating that certain nutrients are being consumed in an optimal way and certain nutrients are being over-consumed. In the embodiment of Fig. 5, the bar that underlays the listed nutrient, amount, and score indicates the *amount* of the nutrient, and the color indicates the value of the score. Specifically, the negative saturated fat score is negative and is therefore displayed in red. The calcium score, on the other hand, is 100 and therefore its score bar is green.

Fig. 5 also illustrates a graphical mechanism for showing the contribution to the overall score of each portion of the meal. Specifically, with regard to calcium, the box behind the French fries column is relatively dark, indicating that the 77% overall intake of calcium originating from French fries makes up a majority of the calcium consumed from the meal. On the contrary, for the tap water column, the majority of the boxes are white to indicate that the tap water is contributing no nutrients for the majority of tracked nutrients. With regard to the hamburger column, the box is relatively dark to indicate that the hamburger contributed 34% of the overall iron consumed in the reflected meal.

In some embodiments, in addition to (or instead of) the individual nutrient scores illustrated in Fig. 5, the disclosed system may calculate and display an aggregate health score as described above. In this embodiment, the same or substantially the same information may be displayed about each of the individual meal components, but rather than pertaining to an individual nutrient, the graphics and colors may indicate the contribution of the meal item to the overall nutritional health score. Thus, for example, an individual can see the extent to which, for example, the hamburger is contributing to the overall score of the meal.

In an embodiment, the score previously defined is used as the basis of a mathematical recommendation system that can be actually implemented in a software tool, helping practitioners and consumers in diet planning. It has to be stressed that a full, user-oriented recommendation engine should also leverage personal preferences, dietary restrictions and take into account personal goals and characteristics like the BMR, energy expenditure, etc. The details of one such software tool are discussed in further detail below.

To illustrate the application of the above Equations to a real-world example, the following example is given using a real meal from the National Health and Nutrition Examination Survey (NHANES) 2011-2012 dataset, illustrated in Table 2 below. NHANES is an ongoing data collection initiative conducted by the National Center for Health Statistics of the CDC. Its purpose to collect information about the health and diet of a cross-sectional, nationally representative sample of the civilian population in the US. In Table 2, the subject is a 27 year old female, 147 centimeters tall, weighing 76.4 kilograms.

**Table 2 - NHANES Example Meal.**

| Food name | Grams |
|---|---|
| Water | 948 |
| Egg casserole with bread, cheese, milk and meat | 189 |
| Milk, cow's, fluid, 1 | 244 |
| Granola bar, NFS | 43 |
| Cheese, Mozzarella, part skim | 28.35 |
| Cheeseburger with tomato and/or catsup, on bun | 114 |
| White potato, French fries, from frozen, deep fried, from fast food / restaurant | 28 |
| Ginger ale | 614 |
| Yogurt, NS as to type of milk or flavor | 245 |
| Berries, raw, NFS | 36 |
| Granola, NFS | 7 |
| Carrots, raw | 50 |
| Cookie, chocolate chip | 16 |
| Cracker, snack | 26.5 |

Table 3 below illustrates the nutrient scores for the various macro-nutrients in the exemplary diet shown in Table 2.

**Table 3 - Scores for Macro-Nutrients**

| Nutrient | Amount | Score |
|---|---|---|
| Ethanol | 0 g | ND |
| Carbohydrate | 260.5 g | 100 |
| Energy | 2144 kcal | ND |
| Fat | 87.2 g | ND |
| Saturated fatty acids | 32.9 g | ND |
| Monounsaturated fatty acids | 28.4 g | ND |
| Polyunsaturated fatty acids | 19.7 g | ND |
| (PUFA+MUFA)/SFA | 1.5 | 23 |
| Protein | 85.6 g | 100 |
| Fiber | 12.4 g | 41 |

Table 4 illustrates the nutrient scores for various minerals in the example diet shown in Table 2.

**Table 4 - Scores for Minerals**

| Nutrient | Amount | Score |
|---|---|---|
| Calcium | 1.6 g | 100 |
| Copper | 1 mg | 100 |
| Iron | 12.3 mg | 97 |
| Magnesium | 252 mg | 94 |
| Potassium | 2304 mg | 53 |
| Sodium | 3084 mg | 66 |
| Zinc | 14.1 mg | 100 |

Table 5 illustrates the nutrient scores for various vitamins in the example diet shown in Table 2.

**Table 5 - Scores for Vitamins**

| Nutrient | Amount | Score |
|---|---|---|
| Folate | 127 mcg | 41 |
| Niacin | 14.5 mg | 100 |
| Riboflavin | 2 mg | 100 |
| Thiamin | 1.3 mg | 100 |
| Vitamin A | 901 mcg | 100 |
| Vitamin C | 35.6 mg | 61 |
| Vitamin D | 6.9 mcg | 72 |
| Vitamin E | 5.8 mg | 49 |
| Vitamin B6 | 1.4 mg | 100 |
| Vitamin B12 | 6.3 mcg | 100 |

With regard to the data illustrated in Tables 2-5, the overall score for the balance of macro-nutrients was 97.5. On the other hand, the global score for the diet was calculated to be 80. This illustrates, in part, that low scores in some micro-nutrients (e.g., sodium) can have a relatively major impact on the overall score calculated according to the disclosure herein.

Referring more particularly now to Figs. 2a, 2b, 2c, 2d, 2e, 2f, 2g, 2h, and 2i, these figures illustrate scoring functions for various nutrients trackable using the disclosed system. In particular, Fig. 2a is the scoring function for calcium, Fig. 2b is the scoring function for copper, Fig. 2c is the scoring function for iron, Fig. 2d is the scoring function for protein, Fig. 2e is the scoring function for vitamin A, Fig. 2f is the scoring function for vitamin B12, Fig. 2g is the scoring function for riboflavin, Fig. 2h is the scoring function for sodium, and Fig. 2i is the scoring function for fiber. It should be appreciated that in various embodiments, the scoring plots illustrated in Figs. 2a, 2b, 2c, 2d, 2e, 2f, 2g, 2h, and 2i are calculated by the system disclosed with regard to Fig. 1 executing steps of algorithms to perform the calculations reflected in the Equations discussed above.

Notably, Figs. 2d (protein), 2f (vitamin B12), 2g (riboflavin), and 2i (fiber) illustrate that regardless of the amount of the respective nutrients consumed, the nutrient score for those nutrients does not decrease as more nutrient is consumed. That is, these scores reflect the fact that overconsumption is not a concern for these nutrients.

On the other hand, for example as shown in Fig. 2h, consuming sodium above the upper limit can eventually cause the sodium score to go to zero. In addition, Figs. 2d and 2i illustrate that the scoring function for protein and fiber depend, in part, on another factor besides simply amount consumed. With regard to Fig. 2d, the scoring function for protein depends, in part, on the body mass of the consumer of protein. With regard to Fig. 2i, the soring function for fiber depends, in part, on the energy consumed by the consumer in a day. These two figures illustrate additional differences in scoring functions for certain nutrients-namely, that for some nutrients additional inputs are needed to determine the nutrient health score for that nutrient.

The disclosed system in various embodiments stores nutrient range consumption values that are as personalized as possible to individual people. The Institute of Medicine publishes recommendations of population-level DRI values for certain nutrients for populations based on gender and age. For example, the Institute of Medicine may specify a handful of recommended nutrient values for infants 0 to 6 months, infants 6 to 12 months, children 1-3 years, children 4-8 years, males 9-13 years, females 9-13 years, and so on. However, these specified values are insufficient for analyzing the overall nutritional health of an individual because they are not actually tailored to individuals. Specifically, they do not take into account a person's health level (*e.g.*, whether the person is athletic or active), health conditions (*e.g.*, obesity, diabetes, allergies), medical limitations (*e.g.*, whether the person is a critical care patient), physiology (height and weight), or any other individual specific consideration. Instead, known recommended intake values are based on age and gender (for certain ages). The system disclosed herein provides a further advantage in that it allows for individuals to have their own, personal, healthy ranges for each nutrient depending on the particular situation for that individual. This personalization can take into account specific condition (*e.g.*, pre-diabetes or cardiovascular issues) or specific goals (*e.g.*, weight loss or weight maintenance).

In further embodiments, individuals may have their own arrangement of weighting values and/or sensitivity values tailored to their own personal health conditions. With these personalized ranges and/or weighting/sensitivity values, the disclosed system can calculate a completely personalized nutritional health score. Indeed, for some individuals, a certain nutrient may not be needed at all, or there may be no negative impact of consuming additional amounts of a nutrient beyond a lower healthy amount of the nutrient. By defining the healthy range of a particular nutrient, the disclosed system can account for that lack of need of the nutrient in a way known systems simply cannot.

In one embodiment, the disclosed system stores a so-called "sensitivity" or "tolerance" value in association with each nutrient tracked by the system. For example, the system may store a data structure or other database of nutrient information containing a "sensitivity" or "tolerance" value for each nutrient. In some embodiments, for some nutrients, no sensitivity value exists, meaning that additional consumption of a nutrient is not harmful. In another embodiment, for some nutrients, the system either assigns an infinite sensitivity value or defines an infinite upper healthy range value to achieve the same outcome--namely, to indicate that overconsumption of a particular nutrient is not harmful.

In various embodiments, the disclosed system stores a plurality of weight values for each nutrient that factors into the nutritional health score H. In general, the larger the weight value for a nutrient, the larger an impact that nutrient will have on the overall nutritional health score. This holds true for under/over consumption as well as healthy consumption. In other words, if a nutrient is being under or over consumed, larger weight values will lower the nutritional health score to a larger extent than smaller weight values. Likewise, if a nutrient is being consumed in a healthy amount, larger weight values will increase the nutritional health score to a larger extent than smaller weight values.

For example, if a nutrient such as "sugar" is given a relatively high scoring weight comparative to vitamins, a food that is low in sugar will in general receive a high nutritional health score. This will be true even if the food is low in other nutrients, as the overall nutritional health score is more dependent on the sugar score. Accordingly, appropriate balancing of weights for different nutrients when personalizing the disclosed system to an individual is of substantial importance.

In various embodiments, the disclosed system enables users to build foods or meals out of sub-foods or components of foods. For example, by indicating to the disclosed system what the user's "normal breakfast" is, the user can store a "breakfast" meal and simply select that one meal when entering an indication of foods he or she plans to eat or has already eaten. That is, the disclosed system enables users to build meals from foods or diets from meals, and to store those multi-component consumables for easy selection and entry at a later time. In some embodiments, the system also enables the user to adjust the amount of a particular multi-component consumable consumed, such as by indicating that the user ate half of his or her normal breakfast.

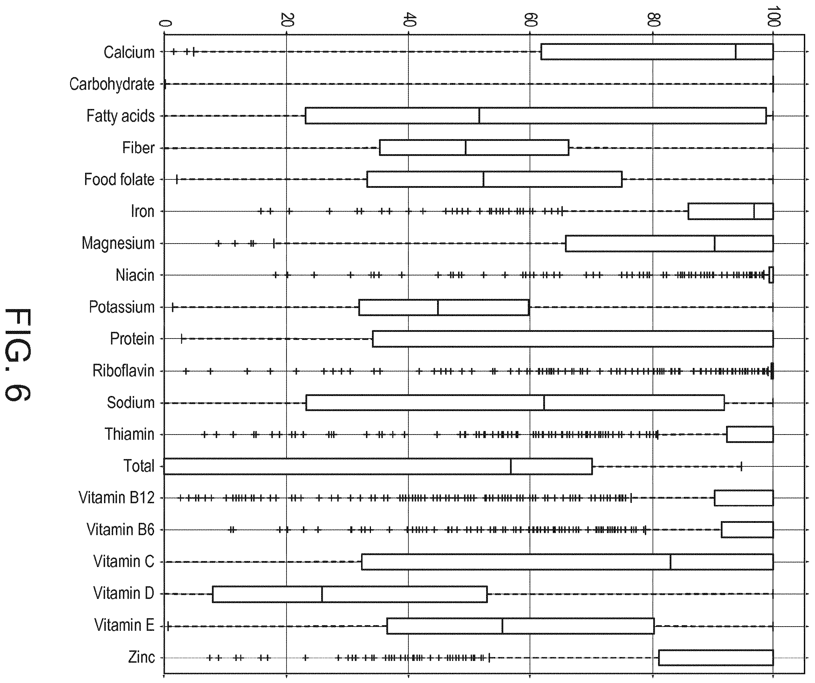

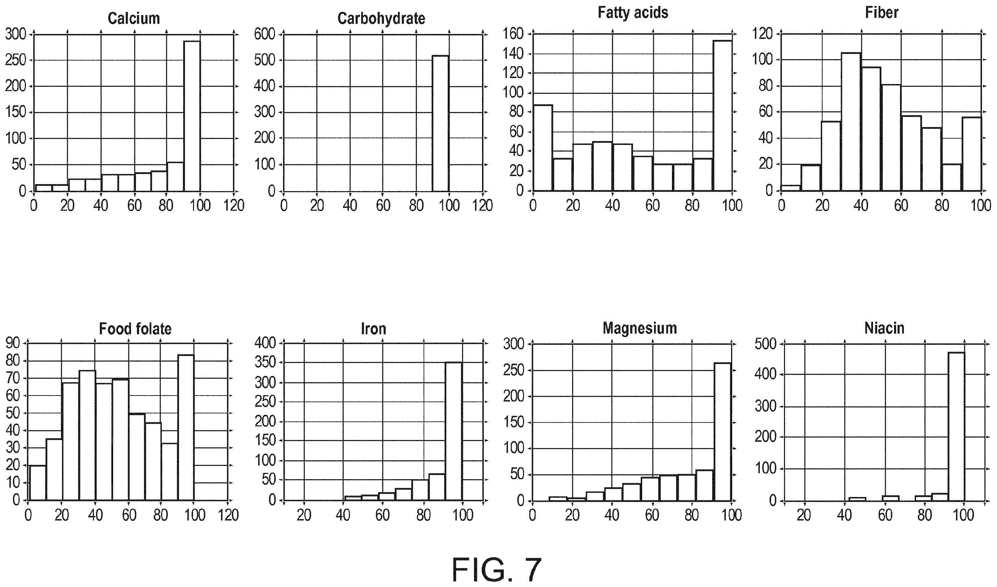

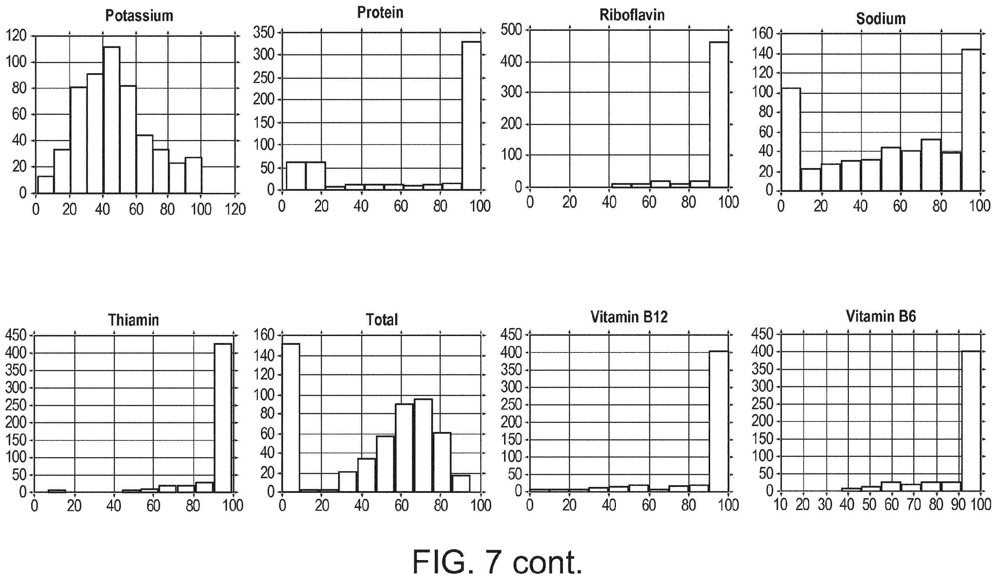

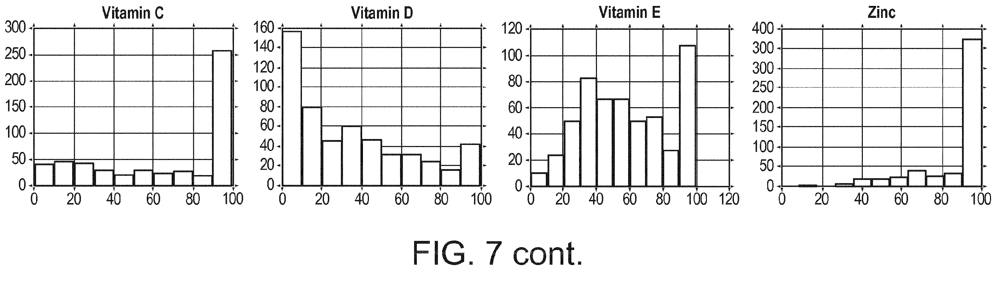

Fig. 6 illustrates a series of box plots of nutritional health scores for a plurality of individual nutrients and an aggregate TOTAL score for the population of females age 31-50 reflected in the NHANES 2011-2012 day 1 data set. Fig. 7 illustrates several individual nutrient score breakdowns for the various individual nutrients tracked in the NHANES data, as well as the TOTAL nutritional health score, as histograms, and supports the box plots of Fig. 6. As can be seen from Figs. 6 and 7, despite the fact that several individual nutrient scores were at the maximal value of 100, none of the TOTAL scores as higher than 80. Likewise, while the number of individual nutrient scores of 0 was relatively low, the number of TOTAL scores of 0 was nonetheless substantially higher (as can be seen by the box extending to a value of 0). This illustrates that overall nutritional health is needed to score a high TOTAL nutritional health score, while low, non-zero individual nutrient scores can lead to a TOTAL nutritional health score of 0.

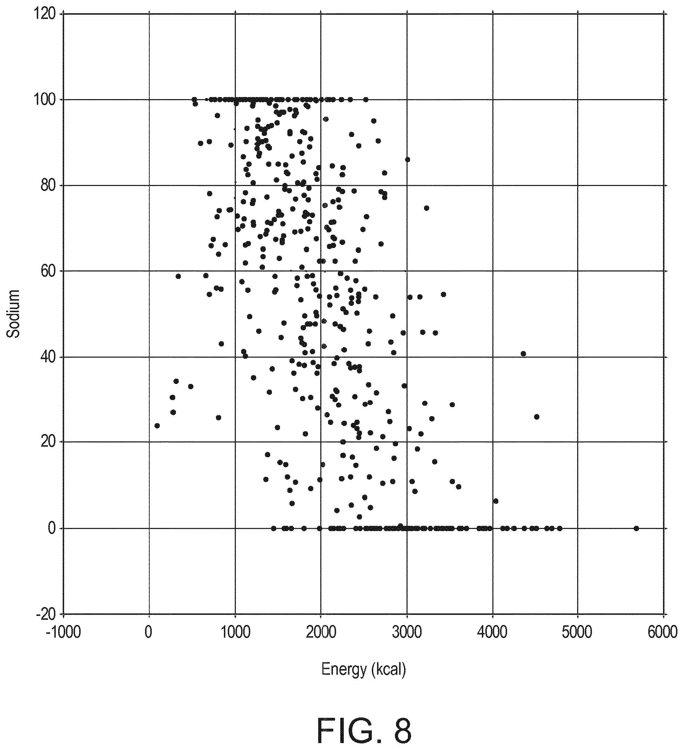

Fig. 8 is a scatter plot illustrating nutrient scores for sodium intake against energy intake in the NHANES 2011-2012 data plotted in Figs. 6 and 7 above. As can be seen in Fig. 8, sodium scores decrease in general as energy intake increases, and eventually, past 3000 kcals, the sodium score drops to 0.

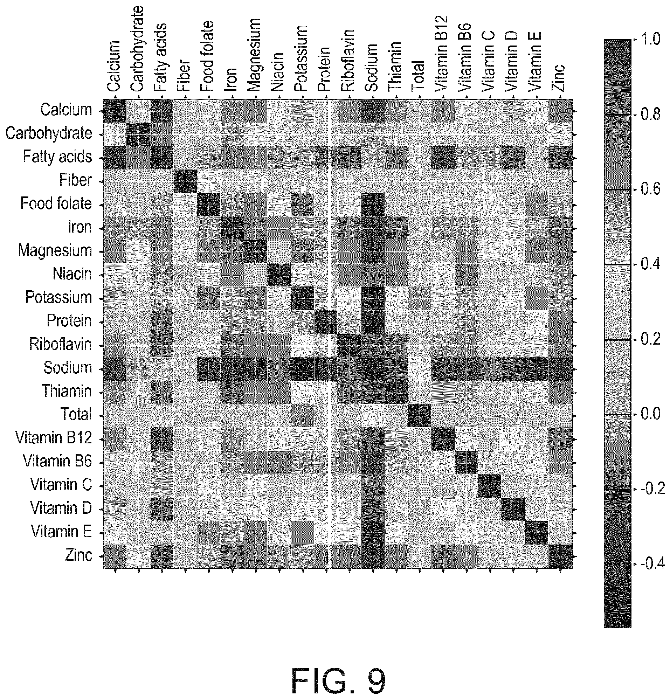

Fig. 9 is a correlation plot illustrating correlations between nutrient scores based on the NHANES 2011-2012 (day 1), Female 31-50 data discussed above. As is apparent from Fig. 9, there was a relatively significant correlation between the Iron and Thiamin scores. However, the total nutritional health score does not appear, from the illustration of Fig. 9, to be significantly correlated to any individual nutrient score. Moreover, the nutrient score for sodium is negatively correlated to all the individual nutrient scores. The score for the fatty acids is also negatively correlated to all the individual nutrient scores. These negative correlations reflect the fact that the consumption of sodium and of saturated fat are generally above the maximum recommended values, meaning that these scores generally drive the remaining scores downward.

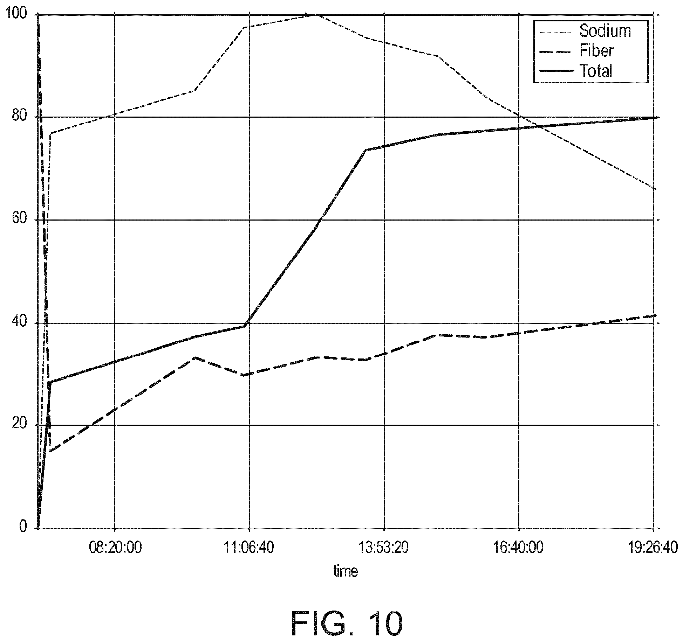

Fig. 10 illustrates a chart showing the evolution of the total score, sodium nutrient score, and fiber nutrient score throughout the course of a day based on data from the NHANES study. In the embodiment shown in Fig. 10, the sodium nutrient score reaches its maximum score at midday, then the intake starts to exceed the maximum recommended daily value and the score decreases. The score for fiber depends on energy intake, therefore the graph can have many local maxima. The scores for the other nutrients (where overconsumption is not harmful) have generally increasing graphs throughout the day.

In various embodiments, the disclosed system may take into account certain "taste" aspects of food when recommending what food to build into a generated diet. For example, if a user indicates he or she would like to have Mexican food for dinner, the system may recommend steak tacos and rice with certain spices to complement the steak tacos. The system in various embodiments also remembers previously recommended foods that the user indicated he or she enjoyed, and tries to recommend foods with similar ingredients or taste profiles. In some embodiments, the system makes recommendations by first trying to select a "main course," such as a chicken breast, and then selecting complementary side items, such as vegetables and rice. In one embodiment, one or more food items are classified by the type of meal they are typically a part of, and recommendations are made based on that classification. For example, scrambled eggs may be classified as a "breakfast" food, and when recommending meals, the system may recommend one or more "breakfast" foods to eat in the mornings.

In one embodiment, the system is configured to integrate with one or more input devices 114 that are personal mobile devices carried by users. For example, a user wearing a pedometer or activity tracker could provide data from those devices to the system, which could adjust the caloric intake range values accordingly. In this way, if a user has a particularly active day, the system may adjust the caloric intake range upward.

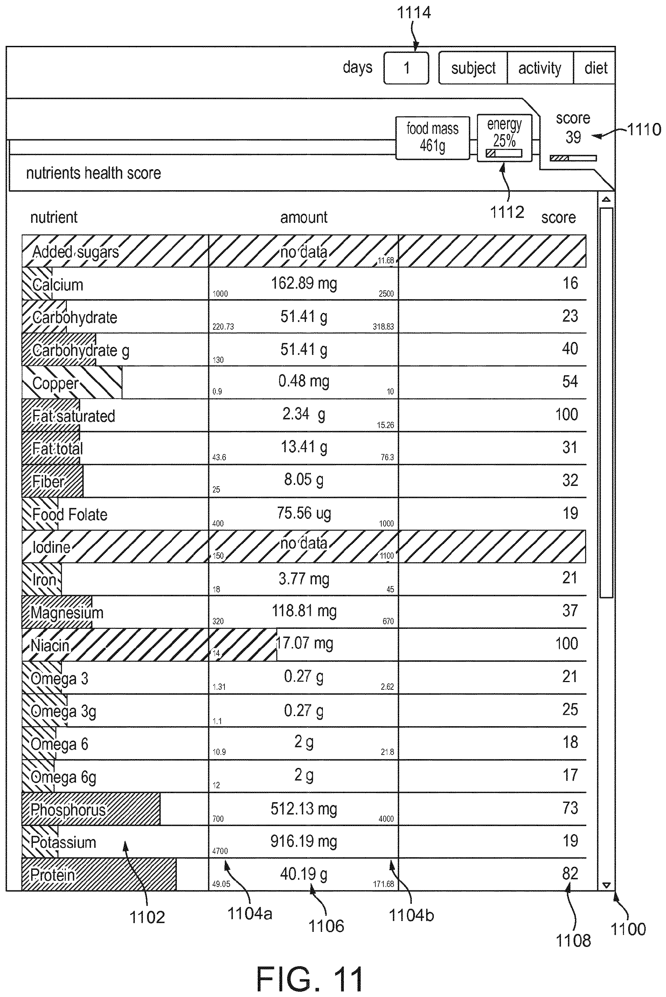

Fig. 11 illustrates an example screen shot of screen 1100 illustrating a plurality of nutrient health scores for a plurality of nutrients consumed by an individual. Fig. 11 illustrates a scoring for a menu sample, considered as a 1-day diet, for a female in the age range 31-50, normal BMI, and having a sedentary lifestyle. The overall score of 39 is quite low; this, however, is expected, as the scoring of Fig. 11 is calculated for a whole day's worth of nutrient consumption, yet the only input values are for a single meal (lunch). It should be appreciated that in Fig. 11, data about some nutrients (*i.e.*, added sugars and iodine) is missing. It should further be appreciated that the ranges displayed for each nutrient correspond to the Recommended Daily Allowance and the Upper Tolerable limit, as defined by the Institute of Medicine.

In the example embodiment of Fig. 11, area 1102 shows the name of each nutrient in the food items consumed by the user; this area also shows a bar illustrating the score for that nutrient. In particular, the bar contained in area 1102 reflects the score illustrated in area 1108. Moreover, in an embodiment, the color of the bar is reflective of whether the score is nearing a maximum score (such as 100). For example, the score for niacin in the example of Fig. 11 is 100 as shown in area 1108. Correspondingly, the bar in area 1102 for niacin is colored in green and is as long as it can be, illustrating a score of 100.

Fig. 11 further illustrates area 1104a and 1104b that indicate a lower and upper intake amount, respectively, for each of the illustrated nutrient. In the illustrated embodiment, the length of the bar for each nutrient illustrates where within the range of minimal to maximal (*i.e.*, 1104a to 1104b) the actually consumed nutrient amounts fall. In addition, Fig. 11 illustrates area 1106 that indicates the amount of each nutrient consumed, and area 1108 contains a numerical representation of what the score is for each consumed nutrient. In addition, area 1114 indicates the amount of time covered by the scores shown in the screen 1100. It should be appreciated that in the illustrated embodiment, the amount of time for the illustrated scores spans a single day; however, the consumption information is for a single meal (lunch). Accordingly the reason many of the scores illustrated in area 1102 score for each of a plurality of nutrients contained in food consumed by the user is that the user has only consumed a single meal, yet the screen 1100 assumes the user has eaten all his or her food for a day.

Fig. 11 also includes area 1110, which illustrates an overall nutritional health score. In the illustrated embodiment, the score is relatively low (39) in large part because the user has only consumed nutrients from a single meal. In addition, area 1112 indicates the amount of energy (*i.e.*, calories) consumed as a percentage of the overall calories to be consumed for the individual over the amount of time indicated in area 1114.

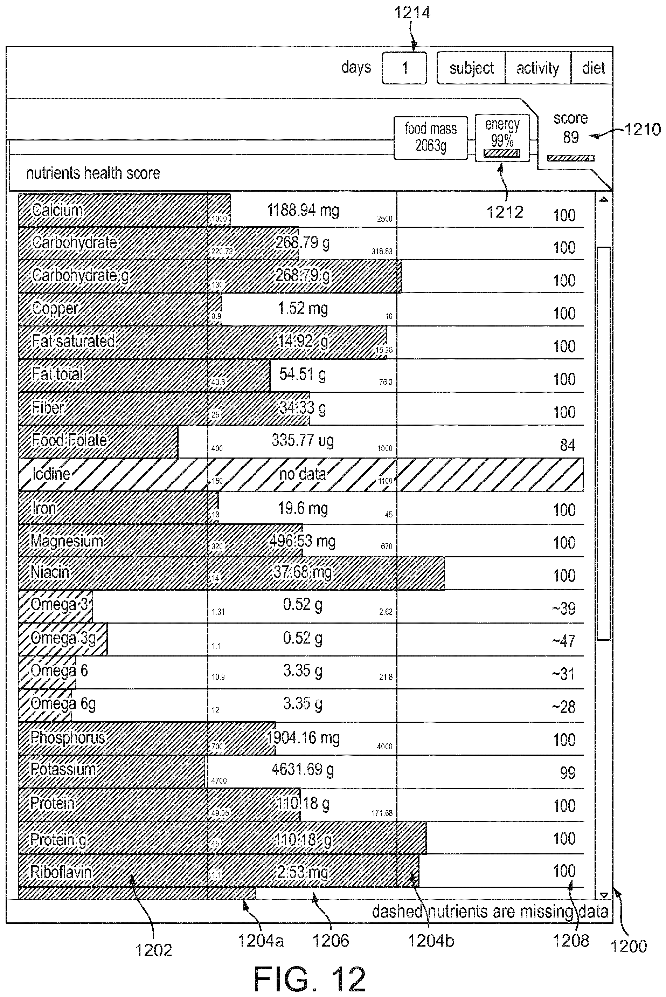

Fig. 12 illustrates a screen shot 1200 similar to the screen shot 1100 of Fig. 11, with nutrient scores for the same subject as in Fig. 11 illustrated. It should be appreciated that despite the correspondence of the areas 1102, 1104a, 1104b, 1106, 1108, 1110, 1112, and 1114 with areas 1202, 1204a, 1204b, 1206, 1208, 1210, 1212, and 1214, respectively, the screen 1200 illustrates nutrient health scores (and overall score in area 1210) for a full day's worth of food consumption. This is apparent from the indication, in area 1212, that the subject has consumed 99% of the recommended daily energy intake. Accordingly, from Fig. 12, it is apparent that the subject has a relatively high overall score of 89 in the course of the day, although has negative scores in Omega nutrients. This indicates that the subject could be consuming a healthier diet if she were to consume more Omega nutrients.

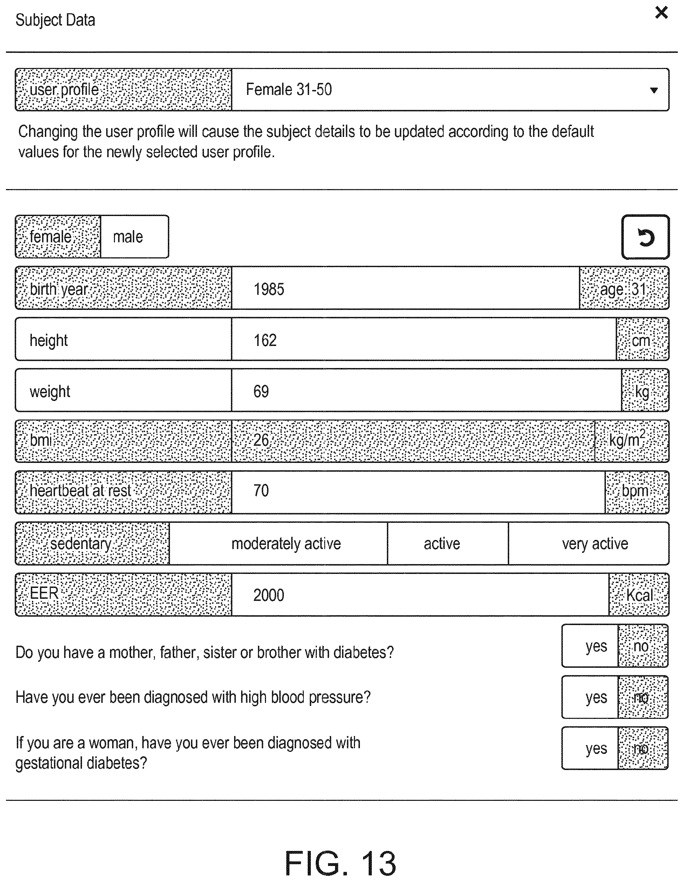

Fig. 13 illustrates a screen shot of a user data entry screen that enables the user to input specific information about himself or herself and enables the system to determine the amount of energy that user requires in a given period of time. In the illustrated embodiment, the EER (Estimated Energy Requirement) calculated by the system as a baseline for calculating nutrient health scores and overall health scores is calculated based on equations promulgated by the Institute of Medicine Equation. In an embodiment, the disclosed system also uses the information inputted in the screen of Fig. 13 to calculate the BMI of the subject individual.

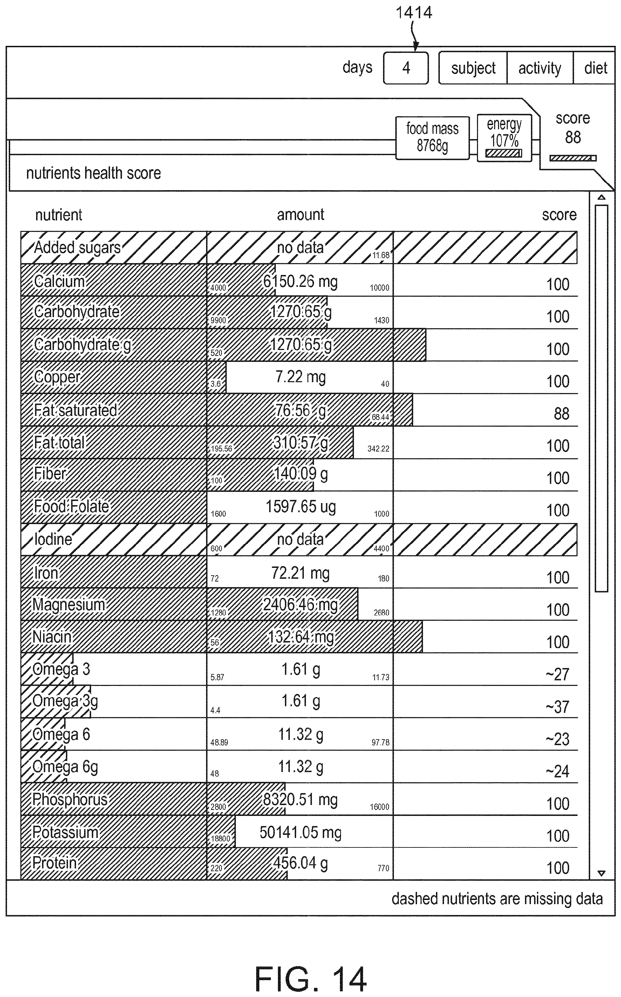

Fig. 14 illustrates a dashboard screenshot similar to the screens of Figs. 11 and 12. However, in area 1414 of Fig. 14, the number "4" has been entered, indicating that the amount of time covered by the scores shown in the screen is 4 days. In the illustrated embodiment, it should be appreciated that the system enables the user to enter the number 4 into area 1414 and thereby adjust the spanned time illustrated in Fig. 14. The scoring algorithms (and minimal and maximal amounts of nutrient intakes) are adjusted accordingly.

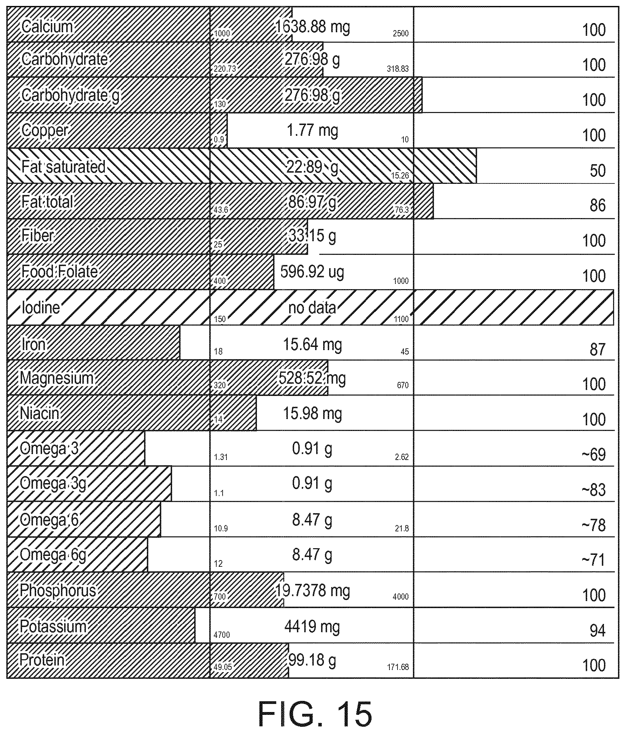

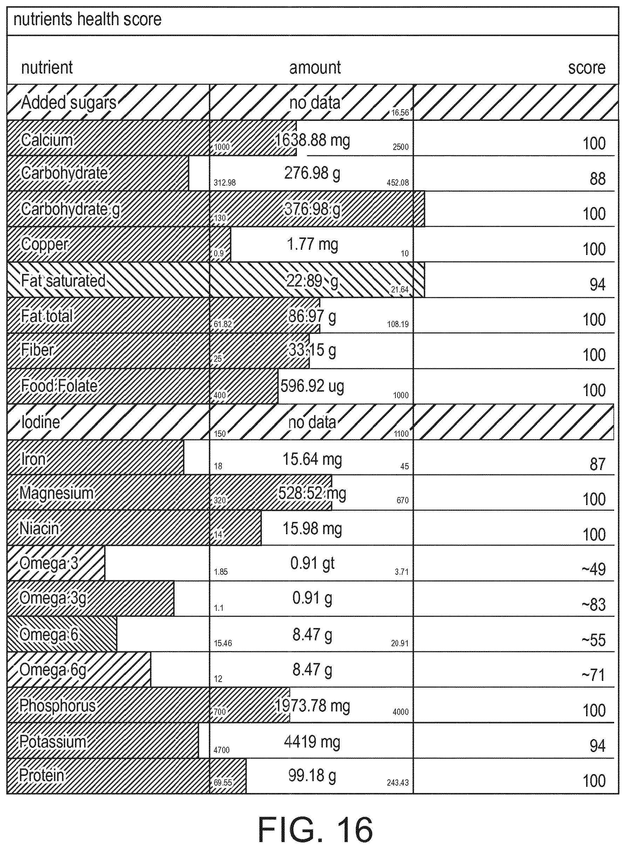

Figs. 15 and 16 illustrate screen shots of an embodiment of the system disclosed herein in which the same foods have been consumed by users with different inputted lifestyles. In particular, in Fig. 15, the user has a sedentary lifestyle, and in Fig. 16, the user has a very active lifestyle. By comparing Fig. 15 with Fig. 16, it can be seen that some of the nutrients (mostly micro-nutrients) have identical recommended intakes (and accordingly identical nutrient health scores). One such nutrient is calcium, which has a recommended intake of 1000 mg regardless of the lifestyle of the subject consuming the nutrient. On the other hand, the recommended protein intake varies between 49.05 and 69.55, respectively, for the sedentary and very active lifestyle individuals. These differences can be further seen with several macronutrients, including saturated fat and carbohydrate. In fact, for these nutrients, the recommended ranges depend on the energy requirements.

In one embodiment, the disclosed system enables a user to determine whether or not to eat foods that do not have complete nutrient content data in the stored database. For example, if a particular entry for syrup in the USDA database does not include stored data indicating how much sugar is present in the syrup, in one embodiment the system displays an icon indicating that data is not present in the database for that syrup. Thus, the system enables a user to see, at a glance, if the score being provided by the system is inaccurate based on a clear lack of data. In the syrup example, if the user's sugar nutrient health score is low, the user may know that the lack of data in what the user knows to be a sugar-rich food is causing the score to appear artificially low.

As used herein the term "consumable" is intended to encompass any item consumed by an individual, such as ingredients, foods, meals, or diets.

In some embodiments, the term "nutrient" as used herein refers to compounds having a beneficial effect on the body e.g. to provide energy, growth or health. The term includes organic and inorganic compounds. As used herein the term nutrient may include, for example, macronutrients, micronutrients, essential nutrients, conditionally essential nutrients and phytonutrients. These terms are not necessarily mutually exclusive. For example, certain nutrients may be defined as either a macronutrient or a micronutrient depending on the particular classification system or list. The expression "at least one nutrient" or "one or more nutrients" means, for example, one, two, three, four, five, ten, 20 or more nutrients.

In various embodiments, the term "macronutrient" is used herein consistent with its well understood usage in the art, which generally encompasses nutrients required in large amounts for the normal growth and development of an organism. Macronutrients in these embodiments may include, but are not limited to, carbohydrates, fats, proteins, amino acids and water. Certain minerals may also be classified as macronutrients, such as calcium, chloride, sodium, or potassium.

In various embodiments, the term "micronutrient" is used herein consistent with its well understood usage in the art, which generally encompasses compounds having a beneficial effect on the body, e.g. to provide energy, growth or health, but which are required in only minor or trace amounts. The term in such embodiments may include or encompass both organic and inorganic compounds, e.g. individual amino acids, nucleotides and fatty acids; vitamins, antioxidants, minerals, trace elements, e.g. iodine, and electrolytes, e.g. sodium chloride, and salts thereof.

In various embodiments, the term "essential nutrient" is used herein consistent with its well understood usage in the art. Essential nutrients are unable to be synthesized internally either at all, or in sufficient quantities, and so must be consumed by an organism from its environment. These include essential fatty acids, essential amino acids, vitamins, and certain dietary minerals. For example, there are two essential fatty acids for humans: alpha-linolenic acid (an omega-3 fatty acid) and linoleic acid (an omega-6 fatty acid). There are nine out of the twenty amino acids that cannot be endogenously synthesized by humans: phenylalanine, valine, threonine, tryptophan, methionine, leucine, isoleucine, lysine, and histidine and these are considered essential amino acids.

In various embodiments, the term "conditionally essential nutrient" is used herein consistent with its well understood usage in the art. Conditionally essential nutrients are certain organic molecules that can normally be synthesized by an organism, but under certain conditions such biosynthesis is not enough to prevent a deficiency syndrome. For example, choline, inositol, taurine, arginine, glutamine and nucleotides are classified as conditionally essential, particularly for neonatal diet and metabolism.

In various embodiments, the term "non-essential nutrient" is used herein consistent with its well understood usage in the art. Non-essential nutrients are those nutrients that can be made by the body; they may often also be absorbed from consumed food. Non-essential nutrients are substances within foods can still have a significant impact on health, whether beneficial or toxic. For example, most dietary fiber is not absorbed by the human digestive tract, but is important in maintaining the bulk of a bowel movement to avoid constipation.

In various embodiments, the term "deficiency" is used herein consistent with its well understood usage in the art. Deficiencies can be due to a number of causes including inadequacy in nutrient intake called dietary deficiency, or conditions that interfere with the utilization of a nutrient within an organism. Some of the conditions that can interfere with nutrient utilization include problems with nutrient absorption, substances that cause a greater than normal need for a nutrient, conditions that cause nutrient destruction, and conditions that cause greater nutrient excretion.

In various embodiments, the term "toxicity" is used herein consistent with its well understood usage in the art. Nutrient toxicity occurs when an excess of a nutrient does harm to an organism.

In various embodiments, the instant disclosure has referenced either databases or datastores as being collections of data. It should be appreciated that depending on the desired implementation, databases (such as databases stored on storage devices operated by the provider of content), datastores (such as cloud computing data storage resources), or other appropriate storage mechanisms could be used to store the various data described herein.

It should be further appreciated that in some embodiments, the amount of food consumed is necessarily determined over a given period of time. For example, a nutritional health score may be one number if a user consumes 1 pound of chicken over the course of a week, and another number altogether if the user consumes 1 pound of chicken in a day. Accordingly, references herein to amounts of food consumed in various embodiments incorporate the concept that the references are to amounts of foods consumed for a given time. Similarly, references to "meals" and "diets" inherently carry with them time period constraints. For example, a meal may be consumed in 1/3 of a day, and a diet may cover a week or a month of time.

As noted above, the disclosed system in some embodiments relies on one or more modules (hardware, software, firmware, or a combination thereof) to perform various functionalities discussed above.

In one embodiment, the disclosed system enables a user to select from among a plurality of databases of potential lower and upper healthy range values, such as by selecting databases of lower and upper healthy range values sourced from different organizations or different countries. For example, in one embodiment the disclosed system enables users to select from databases of healthy range information provided by the USDA on the one hand and governmental bodies in New Zealand on the other hand.

The above description of is exemplary of the features of the system disclosed herein. As noted, the disclosed systems and methods could be used to calculate scores indicating the impact of consumption of consumables on individuals or environmental factors based on any appropriate measurable characteristic of a consumable, and are not limited to determining nutritional value scores. Moreover, the functionality of the above-described system is not limited to the functionalities indicated herein. It should be understood that various changes and modifications to the presently disclosed embodiments will be apparent to those skilled in the art.

**Claims**

1.  A nutritional health score determination system comprising:

   a food input module configured to cause at least one display device to display a food entry control to enable the user to specify at least one food and at least one amount for said at least one food;
   a nutrient health score calculation module configured to determine a minimal intake amount and a maximal intake amount for each of a plurality of nutrients determined in the at least one food,

      the minimal intake amount being based

         i) on a stored Recommended Dietary Allowance (RDA) value and a stored Estimated Average Requirement (EAR) value for the nutrient, and
         ii) if a Recommended Dietary Allowance (RDA) value and an Estimated Average Requirement (EAR) value are not known for a particular nutrient among the plurality of nutrients, on a recommended Adequate Intake (AI) value, and

      to calculate a nutrient health score for each of the plurality of nutrients by:

         (a) determining a first nutrient score function that increases between zero and the determined minimal amount for that nutrient, is flat between the determined minimal amount and the determined maximal amount for that nutrient, and decreases at amounts greater than the determined maximal amount for that nutrient, or by
         (b) determining a second nutrient score function that increases between zero and the determined minimal amount for that nutrient, is flat between the determined minimal amount and the determined maximal amount for that nutrient, and does not decrease at amounts greater than the determined maximal amount for that nutrient,

   wherein the nutritional health score determination system includes a recommendation module configured to operate with the nutritional health score calculation module to determine a plurality of potential nutritional health scores for a plurality of potential consumables and to recommend at least one of the plurality of consumables that results in a highest potential nutritional health score;
   wherein the nutritional health score determination system is further configured:

      i) to enable a user to submit a test to determine the makeup of a bodily fluid of the user, for example blood, urine and/or cerebral fluid, including whether the individual is over- or under-consuming various nutrients among the plurality of nutrients,
      ii) to verify that a recommendation from the recommendation module is working in that the user is actually receiving adequate nutrients when the scoring function indicates that the user's intake ranges are within the desired range, and
      iii) to calibrate itself if the user is receiving too little or too much of a given nutrient, by using the makeup of the user's bodily fluid to alter its scoring algorithms to ensure that a good score, for example 100, actually reflects an ideal intake of that given nutrient for the user.

2. The nutritional health score determination system of Claim **1,** which includes a feedback module configured to calculate a higher nutrient health score using either the first nutrient score function and/or the second nutrient score function based on a different consumed amount of first nutrient(s) and/or second nutrient(s), respectively, and to cause a display of an indication of the higher nutrient health score and the different total consumed amount.

3. The nutritional health score determination system of Claim **2,** wherein the first nutrient is sodium.

4. The nutritional health score determination system of any one of Claims **1-3,** wherein the nutrient health score calculation module is further configured, for a third nutrient, to determine a third nutrient score function that is based on at least one additional input besides the amount of the third nutrient consumed.

5. The nutritional health score determination system of Claim **4,** wherein the at least one additional input includes at least one selected from the group consisting of: body weight and caloric intake.

6. The nutritional health score determination system of Claim **4,** wherein the third nutrient is selected from the group consisting of fiber and protein.

7. The nutritional health score determination system of any one of Claims **1-6,** wherein the nutritional health score calculation module determines an overall nutritional health score by calculating a weighted average of the nutrient health scores for each of the plurality of nutrients.

8. The nutritional health score determination system of Claim **7,** wherein the weighted average is based on at least one characteristic of the user selected from the group consisting of: an activity level of the user, an age of the user, a gender of the user, a weight of the user, a Body Mass Index (BMI) of the user, and a medical condition of the user.

9. The nutritional health score determination system of any one of Claims **1-8,** wherein the recommendation module is configured to cause the at least one display device to display at least one control to enable the user to add the at least one recommended consumable to a diet.

10. The nutritional health score determination system of any one of Claims **1-8,** wherein the recommendation module is configured to cause the at least one display device to display at least one control to enable the user to remove at least a portion of the at least one recommended consumable from a diet.

**Patentansprüche**

1. Bestimmungssystem für eine Ernährungsgesundheitsbewertung, umfassend:

ein Nahrungsmitteleingabgemodul, das konfiguriert ist, um zu bewirken, dass mindestens eine Anzeigevorrichtung eine Nahrungsmitteleintragssteuerung anzeigt, um es dem Benutzer zu ermöglichen, mindestens ein Nahrungsmittel und mindestens eine Menge für das mindestens eine Nahrungsmittel zu spezifizieren;
ein Berechnungsmodul für eine Nährstoffgesundheitsbewertung, das konfiguriert ist, um eine minimale Aufnahmemenge und eine maximale Aufnahmemenge für jeden einer Vielzahl von Nährstoffen, die in dem mindestens einen Nahrungsmittel bestimmt wird, zu bestimmen, wobei die minimale Aufnahmemenge basiert

i) auf einem gespeicherten Wert einer empfohlenen Tagesdosis (RDA) und einem gespeicherten Wert eines geschätzten durchschnittlichen Bedarfs (EAR) für den Nährstoff, und
ii) falls ein Wert der empfohlenen Tagesdosis (RDA) und ein Wert des geschätzten durchschnittlichen Bedarfs (EAR) für einen speziellen Nährstoff unter der Vielzahl von Nährstoffen nicht bekannt sind, auf einem empfohlenen Wert einer angemessenen Aufnahme (AI), und

um eine Nährstoffgesundheitsbewertung für jeden der Vielzahl von Nährstoffen zu berechnen durch:

(a) Bestimmen einer ersten Nährstoffbewertungsfunktion, die zwischen null und der bestimmten minimalen Menge für diesen Nährstoff ansteigt, zwischen der bestimmten minimalen Menge und der bestimmten maximalen Menge für diesen Nährstoff flach verläuft und bei Mengen, die größer als die bestimmte maximale Menge für diesen Nährstoff sind, abnimmt, oder durch

(b) Bestimmen einer zweiten Nährstoffbewertungsfunktion, die zwischen null und der bestimmten Mindestmenge für diesen Nährstoff ansteigt, zwischen der bestimmten minimalen Menge und der bestimmten maximalen Menge für diesen Nährstoff flach verläuft und bei Mengen, die größer als die bestimmte maximale Menge für diesen Nährstoff sind, nicht abnimmt,

wobei das Bestimmungssystem für die Ernährungsgesundheitsbewertung ein Empfehlungsmodul einschließt, das konfiguriert ist, um mit dem Berechnungsmodul für die Ernährungsgesundheitsbewertung zusammenarbeiten, um eine Vielzahl von potenziellen Ernährungsgesundheitsbewertungen für eine Vielzahl von potenziellen Konsumgütern zu bestimmen und um mindestens eines der Vielzahl von Konsumgütern zu empfehlen, das eine höchste potenzielle Ernährungsgesundheitsbewertung ergibt;

wobei das Bestimmungssystem für die Ernährungsgesundheitsbewertung ferner konfiguriert ist:

i) um einem Benutzer zu ermöglichen, einen Test einzureichen, um die Zusammensetzung einer Körperflüssigkeit des Benutzers zu bestimmen, zum Beispiel Blut, Urin und/oder Gehirnflüssigkeit, einschließlich, ob die Person verschiedene Nährstoffe unter der Vielzahl von Nährstoffen über- oder unterkonsumiert,

ii) um zu verifizieren, dass eine Empfehlung aus dem Empfehlungsmodul dabei wirksam ist, dass der Benutzer ausreichende Nährstoffe tatsächlich erhält, wenn die Bewertungsfunktion angibt, dass die Aufnahmebereiche des Benutzers innerhalb des gewünschten Bereichs liegen, und

iii) sich selbst zu kalibrieren, falls der Benutzer zu wenig oder zu viel eines gegebenen Nährstoffs erhält, durch Verwenden der Zusammensetzung der Körperflüssigkeit des Benutzers, um seine Bewertungsalgorithmen zu ändern, um sicherzustellen, dass eine gute Bewertung, zum Beispiel 100, eine ideale Aufnahme dieses gegebenen Nährstoffs für den Benutzer tatsächlich widerspiegelt.

2.  Bestimmungssystem für die Ernährungsgesundheitsbewertung nach Anspruch 1, das ein Rückmeldungsmodul einschließt, das konfiguriert ist, um eine höhere Nährstoffgesundheitsbewertung unter Verwendung entweder der ersten Nährstoffbewertungsfunktion und/oder der zweiten Nährstoffbewertungsfunktion basierend auf einer unterschiedlichen konsumierten Menge an jeweils erstem/ersten Nährstoff(en) und/oder zweitem/zweiten Nährstoff(en) zu berechnen, und um eine Anzeige einer Angabe der höheren Nährstoffgesundheitsbewertung und der unterschiedlichen gesamten konsumierten Menge zu veranlassen.

3.  Bestimmungssystem für die Ernährungsgesundheitsbewertung nach Anspruch **2,** wobei der erste Nährstoff Natrium ist.

4.  Bestimmungssystem für die Ernährungsgesundheitsbewertung nach einem der Ansprüche **1 bis 3,** wobei das Berechnungsmodul für die Nährstoffgesundheitsbewertungs ferner konfiguriert ist, um, für einen dritten Nährstoff, eine dritte Nährstoffbewertungsfunktion zu bestimmen, die auf mindestens einer zusätzlichen Eingabe neben der Menge des konsumierten dritten Nährstoffs basiert.

5.  Bestimmungssystem für die Ernährungsgesundheitsbewertung nach Anspruch **4,** wobei die mindestens eine zusätzliche Eingabe mindestens eine einschließt, die aus der Gruppe ausgewählt ist, bestehend aus: Körpergewicht und Kalorienaufnahme.

6.  Bestimmungssystem für die Ernährungsgesundheitsbewertung nach Anspruch **4,** wobei der dritte Nährstoff aus der Gruppe ausgewählt ist, bestehend aus Ballaststoffen und Protein.

7.  Bestimmungssystem für die Ernährungsgesundheitsbewertung nach einem der Ansprüche **1 bis 6,** wobei das Berechnungsmodul für die Ernährungsgesundheitsbewertung eine Gesamternährungsgesundheitsbewertung durch Berechnen eines gewichteten Durchschnitts der Nährstoffgesundheitsbewertungen für jeden der Vielzahl von Nährstoffen bestimmt.

8.  Bestimmungssystem für die Ernährungsgesundheitsbewertung nach Anspruch **7,** wobei der gewichteten Durchschnitt auf mindestens einer Eigenschaft des Benutzers basiert, die aus der Gruppe ausgewählt ist, bestehend aus: einem Aktivitätsniveau des Benutzers, einem Alter des Benutzers, einem Geschlecht des Benutzers, einem Gewicht des Benutzers, einem Body-Mass-Index (BMI) des Benutzers und einem Gesundheitszustand des Benutzers.

9.  Bestimmungssystem für die Ernährungsgesundheitsbewertung nach einem der Ansprüche **1 bis 8,** wobei das Empfehlungsmodul konfiguriert ist, um zu bewirken, dass die mindestens eine Anzeigevorrichtung mindestens eine Steuerung anzeigt, um es dem Benutzer zu ermöglichen, mindestens ein empfohlenes Konsumgut zu einer Ernährungsweise hinzuzufügen.

10. Bestimmungssystem für die Ernährungsgesundheitsbewertung nach einem der Ansprüche **1 bis 8,** wobei das Empfehlungsmodul konfiguriert ist, um zu bewirken, dass die mindestens eine Anzeigevorrichtung mindestens eine Steuerung anzeigt, um es dem Benutzer zu ermöglichen, mindestens einen Anteil des mindestens einen empfohlenen Konsumguts aus einer Ernährungsweise zu entfernen.

**Revendications**

1. Système de détermination de score de santé nutritionnelle comprenant :

un module d'entrée d'aliments configuré pour amener au moins un dispositif d'affichage à afficher une commande d'entrée d'aliments afin de permettre à l'utilisateur de spécifier au moins un aliment et au moins une quantité pour ledit au moins un aliment ;
un module de calcul de score santé nutritionnelle configuré pour déterminer une quantité d'apport minimal et une quantité d'apport maximal pour chacun d'une pluralité de nutriments déterminés dans l'au moins un aliment, la quantité d'apport minimal étant basée

i) sur une valeur d'apport nutritionnel recommandé (ANR) stockée et sur une valeur de besoin moyen estimatif (BME) stockée pour le nutriment, et
ii) si une valeur d'apport nutritionnel recommandé (ANR) et une valeur de besoin moyen estimatif (BME) ne sont pas connues pour un nutriment particulier parmi la pluralité de nutriments, sur une valeur d'apport suffisant (AS) recommandée, et

pour calculer un score de santé nutritionnelle pour chacun de la pluralité de nutriments par :

(a) la détermination d'une première fonction de score de nutriment qui augmente entre zéro et la quantité minimale déterminée pour ce nutriment, qui est stable entre la quantité minimale déterminée et la quantité maximale déterminée pour ce nutriment, et qui diminue à des quantités supérieures à la quantité maximale déterminée pour ce nutriment, ou par
(b) la détermination d'une deuxième fonction de score de nutriment qui augmente entre zéro et la quantité minimale déterminée pour ce nutriment, qui est stable entre la quantité minimale déterminée et la quantité maximale déterminée pour ce nutriment, et qui ne diminue pas à des quantités supérieures à la quantité maximale déterminée pour ce nutriment ;

dans lequel le système de détermination de score de santé nutritionnelle comporte un module de recommandation configuré pour fonctionner avec le module de calcul de score de santé nutritionnelle afin de déterminer une pluralité de scores de santé nutritionnelle potentiels pour une pluralité de produits consommables potentiels et de recommander au moins l'un parmi la pluralité de produits consommables qui donne lieu à un score de santé nutritionnelle potentiel le plus élevé ;
dans lequel le système de détermination de score de santé nutritionnelle est en outre configuré :

i) pour permettre à un utilisateur de soumettre un test pour déterminer la composition d'un fluide corporel de l'utilisateur, par exemple le sang, l'urine et/ou le liquide cérébral, y compris si l'individu sur- ou sous-consomme divers nutriments parmi la pluralité de nutriments,
ii) pour vérifier qu'une recommandation provenant du module de recommandation fonctionne en ce que l'utilisateur reçoit effectivement des nutriments suffisants lorsque la fonction de notation indique que les plages d'apport de l'utilisateur se situent dans la plage souhaitée, et
iii) pour s'étalonner si l'utilisateur reçoit trop peu ou trop d'un nutriment donné, en utilisant la composition du fluide corporel de l'utilisateur pour modifier ses algorithmes de notation afin de s'assurer qu'un bon score, par exemple 100, reflète réellement un apport idéal de ce nutriment donné pour l'utilisateur.

2. Système de détermination de score de santé nutritionnelle selon la revendication **1,** qui comporte un module de rétroaction configuré pour calculer un score de santé nutritionnelle plus élevé à l'aide soit de la première fonction de score de nutriment et/ou de la deuxième fonction de score de nutriment sur la base d'une quantité consommée

différente de premier(s) nutriment(s) et/ou de second(s) nutriment(s), respectivement, et pour provoquer un affichage d'une indication du score de santé nutritionnelle plus élevé et de la quantité consommée totale différente.

3. Système de détermination de score de santé nutritionnelle selon la revendication **2,** dans lequel le premier nutriment est le sodium.

4. Système de détermination de score de santé nutritionnelle selon l'une quelconque des revendications **1 à 3,** dans lequel le module de calcul de score de santé nutritionnelle est en outre configuré, pour un troisième nutriment, pour déterminer une troisième fonction de score de nutriment qui est basée sur au moins une entrée supplémentaire en plus de la quantité du troisième nutriment consommé.

5. Système de détermination de score de santé nutritionnelle selon la revendication **4,** dans lequel l'au moins une entrée supplémentaire comporte au moins une entrée choisie dans le groupe constitué : du poids corporel et de l'apport calorique.

6. Système de détermination de score de santé nutritionnelle selon la revendication **4,** dans lequel le troisième nutriment est choisi dans le groupe constitué de fibres et de protéines.

7. Système de détermination de score de santé nutritionnelle selon l'une quelconque des revendications **1 à 6,** dans lequel le module de calcul de score de santé nutritionnelle détermine un score de santé nutritionnelle global en calculant une moyenne pondérée des scores de santé nutritionnelle pour chacun de la pluralité de nutriments.

8. Système de détermination de score de santé nutritionnelle selon la revendication **7,** dans lequel la moyenne pondérée est basée sur au moins une caractéristique de l'utilisateur choisie dans le groupe constitué : d'un niveau d'activité de l'utilisateur, d'un âge de l'utilisateur, d'un sexe de l'utilisateur, d'un poids de l'utilisateur, d'un indice de masse corporelle (IMC) de l'utilisateur et d'un problème médical de l'utilisateur.

9. Système de détermination de score de santé nutritionnelle selon l'une quelconque des revendications **1 à 8,** dans lequel le module de recommandation est configuré pour amener l'au moins un dispositif d'affichage à afficher au moins une commande permettant à l'utilisateur d'ajouter l'au moins un produit consommable recommandé à un régime alimentaire.

10. Système de détermination de score de santé nutritionnelle selon l'une quelconque des revendications **1 à 8,** dans lequel le module de recommandation est configuré pour amener l'au moins un dispositif d'affichage à afficher au moins une commande permettant à l'utilisateur de supprimer au moins une partie de l'au moins un produit consommable recommandé d'un régime alimentaire.

FIG. 1

FIG. 2A

FIG. 2B

FIG. 2C

FIG. 2D

FIG. 2E

FIG. 2F

FIG. 2G

FIG. 2H

FIG. 2I

FIG. 3

Scoring percentiles of usual intakes of Calcium

FIG. 4

| nutrient | amount | score | White potato french fries with cheese and bacon | Water tap | Pie lemon cream | Hamburger with tomato and/or catsup on a bun | Breadfruit fried | Chocolate syrup milk added NS as to type of milk |
|---|---|---|---|---|---|---|---|---|
| Alpha carotene | 0 ug | 0 | 0% | 0% | 0% | 0% | 0% | 0% |
| Calcium | 2505.19 mg | 100 | 77% | 3% | 2% | 5% | 1% | 10% |
| Carbohydrate | 250.02 mg | 100 | 22% | 0% | 9% | 13% | 22% | 13% |
| Cholesterol | 263.91 mg | 100 | 44% | 0% | 12% | 12% | 0% | 6% |
| Choline | 230.59 mg | 54 | 42% | 0% | 11% | 15% | 9% | 15% |
| Energy | 2045.68 kcal | 100 | 39% | 0% | 8% | 15% | 13% | 10% |
| Fat saturated | 33.72 g | -21 | 63% | 0% | 5% | 12% | 3% | 8% |
| Fat total | 91.85 g | 80 | 54% | 0% | 7% | 13% | 8% | 5% |
| Fat trans | no data | | n/a | n/a | n/a | n/a | n/a | n/a |
| Fiber | 19.28 g | 77 | 21% | 0% | 1% | 11% | 52% | 4% |
| Food Folate | 186.51 ug | 47 | 29% | 0% | 7% | 26% | 7% | 7% |
| Iron | 9.54 mg | 53 | 24% | 0% | 5% | 34% | 12% | 8% |
| Magnesium | 253.61 mg | 79 | 34% | 10% | 2% | 10% | 20% | 18% |
| Niacin | 13.46 mg | 96 | 33% | 0% | 3% | 34% | 12% | 2% |
| Omega 3 | 1.52 g | 100 | 42% | 0% | 8% | 10% | 27% | 4% |
| Omega 6 | 14.6 g | 100 | 47% | 0% | 11% | 9% | 18% | 1% |
| Phosphorus | 1859.23 mg | 100 | 70% | 0% | 3% | 7% | 3% | 13% |
| Potassium | 2753.12 mg | 59 | 38% | 0% | 2% | 8% | 33% | 14% |
| Protein | 63.68 g | 100 | 49% | 0% | 4% | 24% | 3% | 12% |
| Riboflavin | 1.63 mg | 100 | 39% | 0% | 8% | 13% | 3% | 25% |
| Sodium | 3783.49 mg | 36 | 55% | 3% | 4% | 15% | 13% | 3% |

FIG. 5

FIG. 6

FIG. 7

FIG. 7 cont.

**Vitamin C**

**Vitamin D**

**Vitamin E**

**Zinc**

## FIG. 7 cont.

FIG. 8

FIG. 9

FIG. 10

1114

| days | 1 | subject | activity | diet |

food mass
461g

energy
25%

score
39 — 1110

1112

nutrients health score

| nutrient | amount | | | score |
|---|---|---|---|---|
| Added sugars | | no data | 11.68 | |
| Calcium | 1000 | 162.89 mg | 2500 | 16 |
| Carbohydrate | 220.73 | 51.41 g | 318.83 | 23 |
| Carbohydrate g | 130 | 51.41 g | | 40 |
| Copper | 0.9 | 0.48 mg | 10 | 54 |
| Fat saturated | | 2.34 g | 15.26 | 100 |
| Fat total | 43.6 | 13.41 g | 76.3 | 31 |
| Fiber | 25 | 8.05 g | | 32 |
| Food Folate | 400 | 75.56 ug | 1000 | 19 |
| Iodine | 150 | no data | 1100 | |
| Iron | 18 | 3.77 mg | 45 | 21 |
| Magnesium | 320 | 118.81 mg | 670 | 37 |
| Niacin | 14 | 17.07 mg | | 100 |
| Omega 3 | 1.31 | 0.27 g | 2.62 | 21 |
| Omega 3g | 1.1 | 0.27 g | | 25 |
| Omega 6 | 10.9 | 2 g | 21.8 | 18 |
| Omega 6g | 12 | 2 g | | 17 |
| Phosphorus | 700 | 512.13 mg | 4000 | 73 |
| Potassium | 4700 | 916.19 mg | | 19 |
| Protein | 49.05 | 40.19 g | 171.68 | 82 |

1102   1104a   1106   1104b   1108   1100

FIG. 11

1214

| days | 1 | subject | activity | diet |

food mass
2063g

energy
99%

score
89 ◀——1210

nutrients health score

| | | | | |
|---|---|---|---|---|
| Calcium | 1188.94 mg | 1000 | 2500 | 1212 | 100 |
| Carbohydrate | 268.79 g | 220.73 | 318.83 | | 100 |
| Carbohydrate g | 268.79 g | 130 | | | 100 |
| Copper | 1.52 mg | 0.9 | 10 | | 100 |
| Fat saturated | 14.92 g | 15.26 | | | 100 |
| Fat total | 54.51 g | 43.6 | 76.3 | | 100 |
| Fiber | 34.33 g | 25 | | | 100 |
| Food Folate | 335.77 ug | 400 | 1000 | | 84 |
| Iodine | no data | 150 | 1100 | | |
| Iron | 19.6 mg | 18 | 45 | | 100 |
| Magnesium | 496.53 mg | 320 | 670 | | 100 |
| Niacin | 37.68 mg | 14 | | | 100 |
| Omega 3 | 0.52 g | 1.31 | 2.62 | | ~39 |
| Omega 3g | 0.52 g | 1.1 | | | ~47 |
| Omega 6 | 3.35 g | 10.9 | 21.8 | | ~31 |
| Omega 6g | 3.35 g | 12 | | | ~28 |
| Phosphorus | 1904.16 mg | 700 | 4000 | | 100 |
| Potassium | 4631.69 g | 4700 | | | 99 |
| Protein | 110.18 g | 49.05 | 171.68 | | 100 |
| Protein g | 110.18 g | 45 | | | 100 |
| Riboflavin | 2.53 mg | 1.1 | | | 100 |

dashed nutrients are missing data

1202   1204a   1206   1204b   1208   1200

# FIG. 12

Subject Data                                                                    ✕

| user profile | Female 31-50 | ▼ |

Changing the user profile will cause the subject details to be updated according to the default values for the newly selected user profile.

| female | male | | ↺ |

| birth year | 1985 | age 31 |

| height | 162 | cm |

| weight | 69 | kg |

| bmi | 26 | kg/m$^2$ |

| heartbeat at rest | 70 | bpm |

| sedentary | moderately active | active | very active |

| EER | 2000 | Kcal |

Do you have a mother, father, sister or brother with diabetes?    | yes | no |

Have you ever been diagnosed with high blood pressure?    | yes | no |

If you are a woman, have you ever been diagnosed with gestational diabetes?    | yes | no |

# FIG. 13

1414

| | days | 4 | subject | activity | diet |

food mass
8768g

energy
107%

score
88

nutrients health score

| nutrient | amount | score |
|---|---|---|
| Added sugars | no data 11.68 | |
| Calcium | 4000 6150.26 mg 10000 | 100 |
| Carbohydrate | 9900 1270.65 g 1430 | 100 |
| Carbohydrate g | 520 1270.65 g | 100 |
| Copper | 3.6 7.22 mg 40 | 100 |
| Fat saturated | 7.6.56 g 68.44 | 88 |
| Fat total | 195.56 310.57 g 342.22 | 100 |
| Fiber | 100 140.09 g | 100 |
| Food Folate | 1600 1597.65 ug 1000 | 100 |
| Iodine | 600 no data 4400 | |
| Iron | 72 72.21 mg 180 | 100 |
| Magnesium | 1280 2406.46 mg 2680 | 100 |
| Niacin | 56 132.64 mg | 100 |
| Omega 3 | 5.87 1.61 g 11.73 | ~27 |
| Omega 3g | 4.4 1.61 g | ~37 |
| Omega 6 | 48.89 11.32 g 97.78 | ~23 |
| Omega 6g | 48 11.32 g | ~24 |
| Phosphorus | 2800 8320.51 mg 16000 | 100 |
| Potassium | 18800 50141.05 mg | 100 |
| Protein | 220 456.04 g 770 | 100 |

dashed nutrients are missing data

FIG. 14

EP 3 549 139 B1

| | | | |
|---|---|---|---|
| Calcium | 1000 | 1638.88 mg | 2500 | 100 |
| Carbohydrate | 220.73 | 276.98 g | 318.83 | 100 |
| Carbohydrate g | 130 | 276.98 g | | 100 |
| Copper | 0.9 | 1.77 mg | 10 | 100 |
| Fat saturated | | 22.89 g | 15.26 | 50 |
| Fat total | 43.6 | 86.97 g | 76.3 | 86 |
| Fiber | 25 | 33.15 g | | 100 |
| Food Folate | 400 | 596.92 ug | 1000 | 100 |
| Iodine | 150 | no data | 1100 | |
| Iron | 18 | 15.64 mg | 45 | 87 |
| Magnesium | 320 | 528.52 mg | 670 | 100 |
| Niacin | 14 | 15.98 mg | | 100 |
| Omega 3 | 1.31 | 0.91 g | 2.62 | ~69 |
| Omega 3g | 1.1 | 0.91 g | | ~83 |
| Omega 6 | 10.9 | 8.47 g | 21.8 | ~78 |
| Omega 6g | 12 | 8.47 g | | ~71 |
| Phosphorus | 700 | 19.7378 mg | 4000 | 100 |
| Potassium | 4700 | 4419 mg | | 94 |
| Protein | 49.05 | 99.18 g | 171.68 | 100 |

# FIG. 15

| nutrients health score | | |
|---|---|---|
| nutrient | amount | score |
| Added sugars | no data | |
| Calcium | 1638.88 mg | 100 |
| Carbohydrate | 276.98 g | 88 |
| Carbohydrate g | 376.98 g | 100 |
| Copper | 1.77 mg | 100 |
| Fat saturated | 22.89 g | 94 |
| Fat total | 86.97 g | 100 |
| Fiber | 33.15 g | 100 |
| Food Folate | 596.92 ug | 100 |
| Iodine | no data | |
| Iron | 15.64 mg | 87 |
| Magnesium | 528.52 mg | 100 |
| Niacin | 15.98 mg | 100 |
| Omega 3 | 0.91 gt | ~49 |
| Omega 3g | 0.91 g | ~83 |
| Omega 6 | 8.47 g | ~55 |
| Omega 6g | 8.47 g | ~71 |
| Phosphorus | 1973.78 mg | 100 |
| Potassium | 4419 mg | 94 |
| Protein | 99.18 g | 100 |

## FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016050958 A1

**Non-patent literature cited in the description**

- **GEORGE H. BEATON**. Choice of DRI Value for Use in Nutrition Labeling. *Journal of Nutrition*, 2007
- Dietary Reference Intakes: The Essential Guide to Nutrient. The National Academies Press, 31 December 2006
- *Dietary Reference Intake*, 15 October 2016, https://web.archive.org/web/20161015222358/https://en.wikipedia.org/wiki/Dietary Refe rence Intake